(19) Europäisches Patentamt
European Patent Office
Office européen des brevets

(11) **EP 3 497 601 B1**

(12) **EUROPEAN PATENT SPECIFICATION**

(45) Date of publication and mention
of the grant of the patent:
**16.10.2024 Bulletin 2024/42**

(21) Application number: **17761628.1**

(22) Date of filing: **14.08.2017**

(51) International Patent Classification (IPC):
**G16H 20/60** (2018.01)     **G16H 50/20** (2018.01)
**G16H 50/70** (2018.01)

(52) Cooperative Patent Classification (CPC):
**G16H 20/60; G16H 50/20; G16H 50/70**

(86) International application number:
**PCT/US2017/046728**

(87) International publication number:
**WO 2018/031991 (15.02.2018 Gazette 2018/07)**

(54) **PERSONALIZED NUTRITIONAL RECOMMENDATIONS USING BIOMARKER DATA**

PERSONALISIERTE ERNÄHRUNGSEMPFEHLUNGEN UNTER VERWENDUNG VON
BIOMARKERDATEN

RECOMMANDATIONS NUTRITIONNELLES PERSONNALISÉES AU MOYEN DE DONNÉES DE
BIOMARQUEURS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.08.2016 US 201662374470 P**

(43) Date of publication of application:
**19.06.2019 Bulletin 2019/25**

(73) Proprietor: **Good Measures, LLC
Boston, MA 02109 (US)**

(72) Inventor: **BENNETT, George, B.
Brookline, MA 02445 (US)**

(74) Representative: **Larsen, Charles et al
McDermott Will Emery LLC
22 Bishopsgate
London EC2N 4BQ (GB)**

(56) References cited:
**WO-A1-2015/200898     US-A1- 2012 130 732**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Background

Despite many studies linking nutrition and health, individuals often find it frustrating to improve their overall health. Currently available strategies often result in limited or no success, in part because they rely on inadequate information about the person and his or her objectives, or are focused on only a small number of factors (such as certain nutrients) at a time. Given the complexities and individual variations in human characteristics, it can be very difficult for physicians to provide guidelines that are effective, and very difficult for people to implement the guidelines.

Summary

The correlation between balanced nutrition and wellness is well-established. Numerous studies have confirmed that balanced nutrition can help lower the risk for many chronic diseases, including heart disease, stroke, some cancers, diabetes, and osteoporosis. For example, it has been established that the increased consumption of fruits and vegetables helps reduce the risk for heart disease and certain cancers. Various population studies have also established that certain sub-populations are generally more susceptible to developing certain chronic conditions (e.g., cardiovascular disease, diabetes, cancer, obesity, asthma, etc.) than others. In addition, numerous studies have also classified, diagnosed, quantified, or predicted some or all of these conditions in individuals by using a well-defined set of biomarkers and corresponding biomarker levels. As used herein, a biomarker, or biological marker, generally refers to a measurable indicator of some biological state or condition. A biomarker may be objectively measured and evaluated as an indicator of normal biological processes, pathogenic processes, and/or pharmacologic responses to a therapeutic intervention. As a result, it is possible to quantify the impact of a behavioral change (e.g., diet) or therapeutic intervention by measuring and comparing relevant biomarker levels before and after the behavioral change or therapeutic intervention, in order to determine the effectiveness of the change or intervention.

Such quantifiable results often serve as effective tools for providing feedback to individuals and physicians. With respect to individuals, frequent feedback may improve compliance with a prescribed regimen or behavioral change. However, obtaining such feedback often requires a visit to the treatment center or diagnostic facility, which may be difficult, expensive, inconvenient, and infrequent. In addition, it is also difficult to easily assess a person's progress or the efficacy of the intervention when that assessment involves evaluating multiple biomarker levels, in some cases, more than twenty to fifty. Moreover, a person's diet can have an effect on the efficacy of a medical treatment, in a variety of ways. For example, grapefruit juice may increase the absorption of certain cholesterol-lowering medications, and consuming a large amount of green leafy vegetables may decrease the ability of blood thinners to prevent clotting. Accordingly, close attention to a person's diet while the person is undergoing medical treatment can be advantageous to improving the efficacy of the medical treatment.

U.S. Patent 9,011,153 describes analyzing nutrient intake levels and generating food recommendations that are responsive to a specific user's current nutritional intake and the user's nutrition-related goals. The present disclosure expands on this approach by providing personalized nutrition-related recommendations (e.g., a meal plan) to improve a person's overall health by using the person's biomarker data. The techniques described herein may also be used to provide an aggregate biomarker index that indicates a person's alignment with a target biomarker profile (such as a set of desired levels or ranges for a set of biomarkers, for example), and to provide a predicted change in the index provide the person follows a set of recommendations, including recommended meal plans and/or therapeutic interventions. As is explained in detail below, the systems and methods of the present disclosure may also be used to improve an efficacy of a medical treatment for a particular person. The medical treatment may include a drug, which includes a medicine or any other substance that has a physiological effect when introduced into the body. Improving the efficacy of the medical treatment may include, for example, using biomarker data and other health data (as described below) to assess and recommend diet changes that may reduce the side effect of the treatment (and hence, compliance rates of the medical treatment) and/or enhance or complement the expected therapeutic benefits of the medical treatment. US 2012/130732 A1 discloses a method for personalized nutrition and exercise program and comprehensive wellness assessment. A unique panel of biomarkers from the user's blood or other biological specimen or devices capturing biological/biomedical activities, is assessed and provides information regarding the presence of vitamins, minerals, nutrients, cytokines and other messenger molecules. Biomarker data is collected along with nutrition habits, wellness, physical fitness and exercise regimens" and analysed to provide "optimal food, supplement, life style and exercise recommendation" according to the individual's "personal biology, physiology and personal habit. However said document does particularly not disclose an inference engine configured to isolate a part of the population who experienced similar medication effects as the person in question and identifies a segment of the part of the population that exhibited most improved biomarker indexes, and generate a recommended meal plan by assigning as a candidate nutrient template the nutrient template assigned to the segment of the part of the population that exhibited most improved biomarker indexes. The present application discloses a system for providing a personalized meal plan for improving an efficacy of a medical treatment for a person, the system comprising:one or more data storage devices for storing one or more electronic knowledge bases comprising (i) health data collected from a plurality of data sources and related to a plurality of demographics, (ii) medical data for a plurality of medical treatments, and (iii) nutritional data including a plurality of meals previously consumed by the person and a library of nutrient contents for a plurality of nutrient templates; an inference engine implemented on one or more processors including hardware and software, the inference engine being configured to:(a) receive biomarker data indicative of a measured biomarker level for each of a plurality of biomarkers for the person,(b) receive medical treatment data indicative of one or more medications to be administered to the person,(c) deduce, using an artificial intelligence technique using machine learning, based on the medical treatment data, the biomarker data, the health data, and the medical data from the knowledge base, a plurality of queries associated with a plurality of effects of the one or more medications, the queries comprising a question of whether or not the person has observed an effect during the medical treatment or the administration of the one or more medications, (d1) receive a plurality of inputs indicative of responses to the queries, (d2) based on the plurality of responses to the queries, isolate in the one or more electronic knowledge bases a data set indicative of a part of the population who experienced similar effects as the person, and identify a segment of the data set indicative of a part of the population that exhibited most improved biomarker indexes, and(e) generate by the one or more processors, based on the inputs, a recommended meal plan by assigning as a candidate nutrient template the nutrient template assigned to the segment of the part of the population that exhibited most improved biomarker indexes, and modifying the candidate nutrient template, wherein the modifying is based on a subset of the previously consumed meals and comprises subtracting nutrient amounts consumed by the person over a time period prior to generation of the meal plan to obtain an updated target nutritional profile, and the recommended meal plan is designed to reduce a negative effect of the one or more medications, increase a beneficial effect of the one or more medications and/or minimize a deviation from a target biomarker template for the person;f) generate, based on the recommended meal plan, a predicted biomarker index indicative of an aggregate alignment between the target biomarker template and the biomarker data for the person upon following the recommended meal plan for a predetermined time period, and a communications port for providing the recommended meal plan to a user interface and display the meal plan and the predicted biomarker index to the person for selection by the person.

In some implementations, the inference engine is further configured to (g) generate, based on the biomarker data, a biomarker index indicative of an aggregate alignment between the target biomarker template and the biomarker data for the person, and the communications port is further for providing the biomarker index to the person. The biomarker index may be based on a weighted function of deviations between target biomarker levels defined by the target biomarker template and measured biomarker levels in the biomarker data for the person. The weighted function may include a biomarker coefficient applied to the deviation for each respective target biomarker, the biomarker coefficient being indicative of a relative importance of the respective target biomarker to other biomarkers in the target biomarker template for the one or more medications.

In some implementations, the health data includes biomarker data for a plurality of persons who were previously administered the one or more medications and the corresponding nutrients consumed by the plurality of persons during the period of time corresponding to the administration of the one or more medications.

In some implementations, the plurality of queries corresponds to common side effects associated with the one or more medications.

In some implementations, the negative effect corresponds to one or more undesirable side effects that negatively impact efficacy of the one or more medications or negatively impacts compliance with a prescribed regimen of the one or more medications. The one or more undesirable side effects may include at least one of bloating, gas, nausea, vomiting, diarrhea, tiredness, constipation, weight gain, weight loss, and any combination of two or more of the foregoing.

In some implementations, the beneficial effect corresponds to one or more target therapeutic effects of the one or more medications.

In some implementations, the target biomarker template is determined based on consensus guidelines regarding target biomarker ranges for a healthy individual, the person's demographic information, and the medical treatment. The demographic information may include genetic information obtained based on a biological sample of the person.

In some implementations, the one or more electronic knowledge bases further comprise (iv) clinical data related to a clinical testing setting, wherein the inference engine is further configured to override an input from the person with respect to the one or more medications if the input conflicts with one or more inputs derived from the clinical data.

In some implementations, the system further comprises a user interface for a clinician associated with the person, the user interface including a dashboard having options to display information related to the person, including the person's demographic information, the health data related to the person's demographics, the measured biomarker levels for the person, the one or more medications to be administered to the person, amounts of nutrients in the plurality of meals previously consumed by the person, and the recommended meal plan.

In some implementations, the system further comprises a user interface for the person, the user interface including options to display information related to the person, including the person's demographic information, the measured biomarker levels for the person, the one or more medications to be administered to the person, amounts of nutrients in the plurality of meals previously consumed by the person, and the recommended meal plan.

In some implementations, the plurality of medical treatments include treatments for medical conditions including intestinal disorders, cardiovascular disease, cancer, obesity, diabetes, and allergies.

In some implementations, the system further comprises a user input device for manually receiving entries related to the plurality of meals previously consumed by the person and the one or more medications.

In some implementations, the system further comprises a data receiving module for automated input of data indicative of the plurality of meals previously consumed by the person for storage in the one or more data storage devices, without input from the person.

In some implementations, the data indicative of the one or more medications includes at least a dose and a time of administration for each administration of the one or more medications.

In some implementations, the measured biomarker levels for the plurality of biomarkers are measured from a sample of the person's blood.

In some implementations, the recommended meal plan is recommended for a predetermined period of time. The predetermined period of time is determined based on a duration of the medical treatment. The invention is defined by the appended claims.

Brief Description of the Drawings

The above and other features of the present disclosure, including its nature and its various advantages, will be more apparent upon consideration of the following detailed description, taken in conjunction with the accompanying drawings in which:

FIG. 1 is a block diagram of a computerized system for providing personalized nutritional assessment and recommendations that are responsive to a person's health-related goals, according to an illustrative implementation.
FIG. 2 is a flow chart of a method used by a computerized system to provide a personalized meal plan for improving an efficacy of a medical treatment for a person, according to an illustrative implementation.
FIG. 3 is a flow chart of a method used by a computerized system to identify a nutrient template that is predicted to perform best for a group of people, according to an illustrative implementation.
FIG. 4 is a flow chart of a method used by a computerized system to assess a biomarker index, according to an illustrative implementation.
FIG. 5 is a block diagram of a computing device for performing any of the processes described herein, according to an illustrative implementation.

Detailed Description

Described herein are nutrition analysis and recommendation techniques for providing personalized nutritional assessment and recommendations that are responsive to the person's health-related goals. The goals may include those related to the therapeutic efficacy of medical treatments. In particular, the methods and systems described herein are suitable for assessing the efficacy of a medical treatment as measured by its impacts on various biomarkers and recommending foods that improve the efficacy of the medial treatment by, for example, enhancing the therapeutic effects or reducing undesirable side effects.

To provide an overall understanding, certain illustrative implementations will now be described, including a system for suggesting foods to help a user modulate his or her nutrient consumption to meet personalized goals, such as improving the efficacy of a medical treatment. However, it will be understood by one of ordinary skill in the art that the systems and methods described herein may be adapted and modified as is appropriate for the application being addressed and may be employed in other suitable applications, and that such other additions and modifications will not depart from the scope thereof.

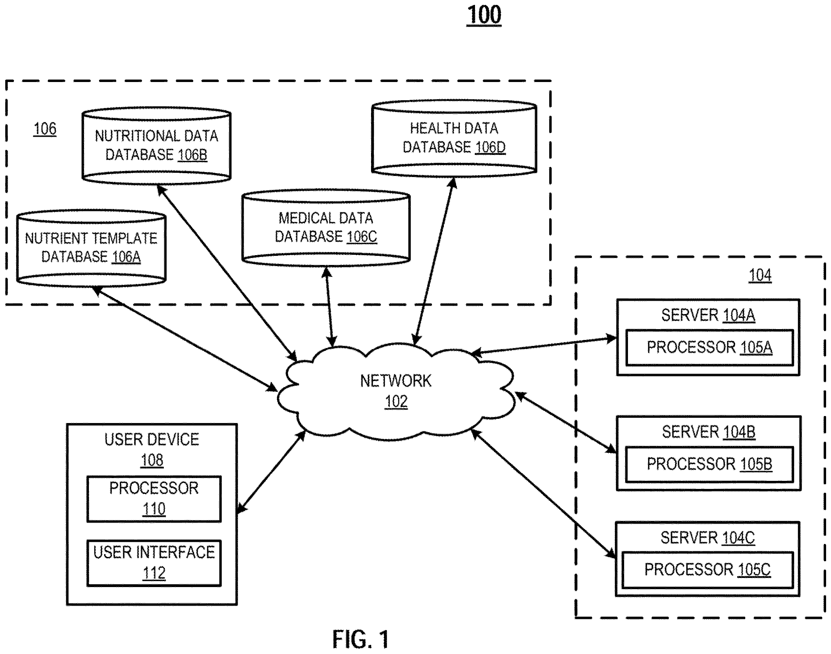

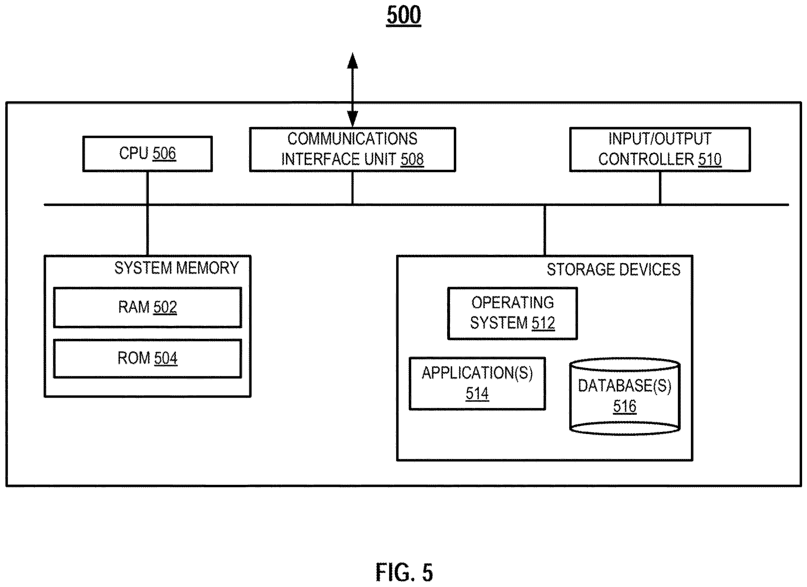

FIG. 1 depicts a system 100, an example of a network and database structure, that may be used to implement the systems and methods disclosed herein. For example, the computerized system 100 can be used to modulate a person's diet to improve an efficacy of a medical treatment for the person, according to an illustrative implementation. Additionally, or alternatively, the system 100 may be used to provide an aggregate indicator of a person's overall health, using the person's biomarker levels. The aggregate indicator may a single indicator (e.g., a number or a grade) that provides an indication of an alignment between the person's biomarker levels and a target biomarker profile. Rather than simply representing a single biomarker at a time, the aggregate indicator represents multiple biomarkers simultaneously, and may represent any number of biomarkers, such as 2, 3, 4, 5, 10, 15, 20, 30, 40, 50, or any other number of biomarkers. The system 100 includes a server 104 and a user device 108 connected over a network 102 to the server 104. The user device 108 includes a processor 110 and a user interface 112. As used herein, the term "processor" or "computing device" refers to one or more computers, microprocessors, logic devices, servers, or other devices configured with hardware, firmware, and software to carry out one or more of the computerized techniques described herein. Processors and processing devices may also include one or more memory devices for storing inputs, outputs, and data that is currently being processed. An illustrative computing device 500, which may be used to implement any of the processors and servers described herein, is described in detail below with reference to FIG. 5. As used herein, "user interface" includes, without limitation, any suitable combination of one or more input devices (e.g., keypads, touch screens, trackballs, voice recognition systems, *etc*.) and/or one or more output devices (*e.g.*, visual displays, speakers, tactile displays, printing devices, *etc*.). As used herein, "user device" includes, without limitation, any suitable combination of one or more devices configured with hardware, firmware, and software to carry out one or more of the computerized techniques described herein. Examples of user devices include, without limitation, personal computers, laptops, and mobile devices (such as smartphones, blackberries, PDAs, tablet computers, *etc*.).

As depicted in FIG. 1, the components of the system 100 are distributed over multiple processing and storage devices connected via the network 102. Such an implementation may be appropriate for distributed computing over multiple communication systems including wireless and/or wired communication systems that share access to a common network resource. In some implementations, the system 100 is implemented in a cloud computing environment in which one or more of the components are provided by different processing and storage services connected via the Internet or other communications system. The server 104 is an inference engine implemented on at least one processor including hardware and software, and is configured to perform any of the processes described herein. As depicted in FIG. 1, the server 104 includes a distributed system of servers that includes server instances 104A, 104B and 104C, each including processor instances 105A, 105B and 105C, respectively. The server instances 104A, 104B and 104C may be, for example, virtual servers instantiated in a cloud computing environment. Any function described herein as being performed by the server 104 or the processor 105 may be performed by any one or any combination of the server instances or processor instances. Three server instances and one user device are shown in FIG. 1, but the system 100 can support any number of servers and any number of user devices. While the server 104 is depicted as a distributed system of server instances, the server 104 may not be configured as a distributed system and may only include a processor 105.

The database 106 is a distributed system of databases that includes a "nutrient template" database 106A, a "nutritional data" database 106B, a "medical data" database 106C, and a "health data" database 106D. Any combination of the databases 106A-106E (or other databases described below but not shown in FIG. 1) may be combined into a common database.

The nutrient templates database 106A stores target levels of nutrients, in accordance with different dietary programs, and for different demographics. A dietary program may relate to one or more specific nutrition-related goals, and may be based on particular desired health outcomes. For example, a person's dietary program may manage specific medical or dietary conditions while meeting nutrient needs for the person. The dietary program may also consider the person's food preferences, such as a desire to try new foods, consume more fruits and vegetables, adhere to a specific diet (such as a low carbohydrate diet or diets targeted towards a person of the weight, age, gender, and overall health of the person, for example), adhere to U.S.D.A. guidelines, or adhere to any other nutritional program aimed to improve or maintain the person's overall health. Dietary programs are explained in further detail in U.S. Patent 9,011,153. A demographic relates to information describing a person and may include genetic information that is obtainable from a biological sample of the person. In an example, the demographic of the person includes the person's age, weight, height, sex, ethnicity, body fat percentage, medical condition, or any other information that describes a person or a trait of the person that is shared by others.

The data stored in the nutrient template database 106A corresponds to target levels of nutrients in accordance with one or more selected dietary programs. This data may also be referred to as a target nutritional profile. The target nutritional profile may be specifically generated for a particular person, and may be dependent on the person's health and demographic features. The target nutritional profile may correspond to the person's dietary program (determined, for example, to achieve the person's nutrition-related goal), or to a predetermined target. In an example, a person may have a specific medical condition that requires his consumption of a certain nutrient to be below a certain level. In another example, a person's desire to adhere to a low carbohydrate may restrict the person's target nutritional profile to include a specific target level or range of carbohydrates that is lower than would otherwise be recommended for the person.

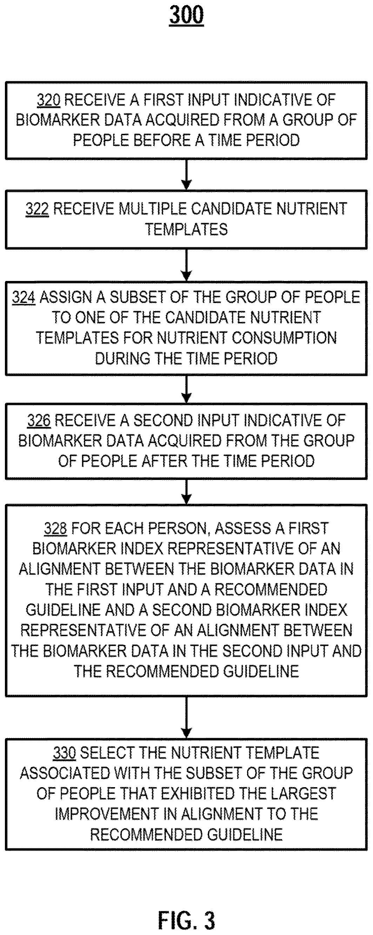

The set of nutrient templates stored in the nutrient templates database 106A may be identified based on technical or scientific literature, such as peer-reviewed publications. For example, the literature may include recommended guidelines for various target nutritional levels for people with particular demographics. By parsing the literature to identify potentially optimal target nutritional profiles for certain segments of the population, the number of subsets of the group of people used in studies resembling that shown in FIG. 3 may be reduced. In particular, rather than testing a group of people with the set of all possible nutrient templates, the set of nutrient templates may be reduced to only include those that are likely to improve the health of a person with certain demographic data.

In some implementations, a nutrient template includes multiple target nutritional profiles, each of which may be a variant or a modification of a first target nutritional profile. For example, the first target nutritional profile may include target nutrient levels that are tailored for a Caucasian male adult of an average height and weight. The nutrient template corresponding to the first target nutritional profile may further include variants of that first profile that are specifically tailored for people with different demographics. For example, the nutrient template may include a second target nutritional profile that is tailored for a Caucasian female adult of an average height and weight. The first and second profiles may be similar, and the second profile may simply be stored in the nutrient templates database 106A as a slight variant of the first profile (by reducing all of the target nutrient levels by a certain percentage, for example). Similarly, the nutrient template may include other target nutritional profiles that are tailored for people fitting other demographic features, such as Caucasian males or females with different heights or weights, children, persons with different ethnicities, ages, or medical conditions. In this manner, a single nutrient template may include multiple target nutritional profiles, each of which may be defined for a different segment of the population. In an example, a single nutrient template may include a single target nutritional profile, but may further include instructions for how to modify the single target nutritional profile to accommodate people with various demographics.

The nutritional data database 106B stores consumed levels of nutrients for a person who may be interacting with the system 100 over the user device 108. A nutrient includes any source of nourishment, typically found in food, but may also be found in other sources or produced internally by the body. Examples of nutrients include calories; proteins or their building blocks, such as amino acids; water; carbohydrates including monosaccharides such as glucose, disaccharides such as sucrose, and oligosaccharides and polysaccharides such as starch, glycogen, and cellulose; lipids, fatty acids, and other fats; fiber; cholesterol; omega-3; vitamins and dietary or nutritional supplements; potassium; calcium; magnesium; nitrates; phosphates; iron; zinc; other elements such as copper; sodium; minerals; or any other nutrient.

The person reports the foods and/or nutrient amounts that the person consumes by entering food log entries into the user interface 112 or instructing or authorizing another person (such as a clinician, a dietician, a restaurant or food provider, for example) to enter such food log entries. Such food log entries are described in detail in U.S. Patent 9,011,153 and may be manually entered by the person, or automatically input into the system 100 without input from the person. Briefly, the food log entry may include a name of the food consumed or about to be consumed, a time, date, and/or location that the food is consumed, as well as the nutritional content of those foods. Food includes any substance consumed to provide nutritional support for the body, and typically includes nutrients that are absorbed by the body to stimulate growth, maintain life, or provide energy. Food may include substances of man-made or natural origin, and may be consumed in their naturally occurring form (e.g., an apple, an orange, a potato, beef) or in prepared or packaged forms including one or more other foods (e.g., a meal, an energy bar, or a beverage).

The medical data database 106C stores data related to multiple medical treatments. The medical treatments include treatments for medical conditions that may be directly or indirectly related to a person's nutrition and includes medical conditions that are affected by the person's diet and/or that affect the person's diet. Such medical conditions may include as intestinal disorders, cardiovascular disease, cancer, obesity, diabetes, allergies, any combination of these, etc. A medical treatment may be a course of action undertaken by a patient or a clinician in order to heal the patient from the medical condition, prevent a medical condition from occurring or worsening, alleviate symptoms associated with the medical condition, or any combination thereof. Examples of medical treatments include administration of one or more medications (including drugs taken orally, intravenously, intramuscularly, intrathecally, subcutaneously, sublingually, buccally, rectally, vaginally, ocularly, oticly, nasally, by inhalation, by nebulization, cutaneously, topically, or transdermally), surgery, physical therapy, acupressure, acupuncture, aromatherapy, chiropractic, homeopathy, reflexology, or any other suitable method of treating someone with a medical condition.

The data stored in the database 106C may further include data that is descriptive of various parameters of the medical treatment, such as dosage, concentration level, frequency, and duration of the medical treatment, as well as various modifications of such parameters for patients of different demographics, such as children versus adults, or based on the patient's weight, sex, or height. In an example, the data stored in the database 106C may further include effects data that is descriptive of a set of possible effects as a result of each medical treatment. In particular, such side effects may include intended positive consequences of the medical treatment and/or unintended negative consequences of the medical treatment. The positive consequences of the medical treatment include beneficial effects, such as target therapeutic effects of the medical treatment, while the negative consequences of the medical treatment include undesirable side effects that negatively impact the efficacy of the medical treatment and/or negatively impacts compliance with a prescribed regimen of the medical treatment. Such undesirable side effects may include bloating, gas, nausea, vomiting, diarrhea, tiredness, constipation, weight gain, weight loss, or any other suitable undesirable effect.

The health data database 106D stores data related to multiple demographics in a population of people and is used to mine information useful for providing nutritional recommendations to a person. Specifically, the data stored in the health data database 106D is used as a reference data set to predict an expected improvement to the overall health of the person if the person consumes foods in accordance with a particular nutrient template. The overall health of the person may be assessed according to the person's biomarker data, which is described in detail below with reference to a "biomarker index". The health data includes data related to the health of a population of people, and includes medical treatment data related to one or more medical treatments used to treat people in the population, nutritional data related to nutrient amounts consumed by people in the population, and biomarker data obtained from people in the population. As described herein, the biomarker data may be used to assess an overall health of a person, and an increased alignment between a person's measured biomarker data and biomarker guidelines may be an indicator of an improved overall health of the person. In an example, the biomarker data may include the data shown below in column A of Table 1.

The health data in the health data database 106D may be collected from multiple data sources. The multiple data sources that provide the health data include private companies, public agencies, or a combination of both private companies and public agencies, and may be the result of one or more extensive clinical studies where the medical treatments, diets, and biomarker data of a population of people are closely monitored. An example method of how the data stored in the health data database 106D is generated is described in relation to FIG. 3. Essentially, a population of people is divided into multiple portions of the population. Different portions of the population undergo different medical treatments to treat or prevent certain medical conditions. Then, each portion of the population is further divided into multiple subsets of people. Each subset is assigned to consume foods in accordance with a nutrient template, such as any of the nutrient templates stored in the database 106A. The biomarker data of the population of people are compared to biomarker target levels at multiple times during a time period during which the medical treatments are administered to the population and during which their nutritional diets are monitored. The result is a large database of health data that may then be used to provide nutritional recommendations to a user. In particular, the health data is analyzed to identify the portion of the population that has similar demographics as the user and underwent the same medical treatment that the user is undergoing or about to undergo. Then, the nutrient template for the subset of the portion of the population that exhibited the most improved or most aligned biomarker data to the biomarker target, is identified as the optimal nutrient template. The optimal nutrient template is then used for providing nutritional recommendations to the user for optimally improving an efficacy of the medical treatment for the user, such as by reducing a negative effect of the medical treatment, by increasing a beneficial effect of the medical treatment, or by minimizing a deviation from a target biomarker template for the user.

The medical treatment data stored in the health data database 106D may include data that is stored in the medical data database 106C, and may further include specific information regarding the medical conditions and medical treatments for the people in the population. The nutritional data stored in the health data database 106D includes data similar to that stored in the nutritional data database 106B, but applicable for the people in the population.

The biomarker data stored in the health data database 106D may include physiological measurements taken from a person, including without limitation blood test results, lipid panel test results, cholesterol screening results, psychological test results, fitness test results, cardiovascular assessments, side effects of a drug, or any other suitable physiological assessment that may be measured or acquired from a person in the population. The biomarker data may further include biomarker index data that provides an indication of a person's overall health. The biomarker index data may be specifically related to a medical treatment applied to the person, and may represent a deviation from a target biomarker template for the person, an aggregate negative effect of the medical treatment, an aggregate beneficial effect of the medical treatment, or a suitable combination thereof.

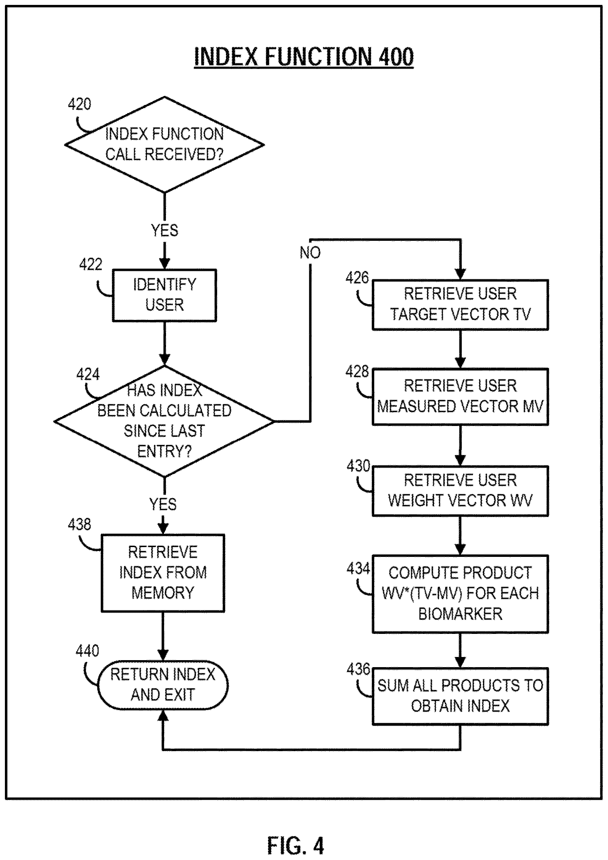

In one example, the biomarker index is based on a person's measured biomarker levels ("the measured biomarker profile") and target biomarker levels (the "target biomarker profile"). As used herein, the phrase "based on" means "based at least in part on". The target biomarker profile includes a set of target biomarker levels or ranges (e.g., one target biomarker level or range for each biomarker in a set of biomarkers), and is determined according to a recommended consensus guideline for the general public (e.g., the target biomarker levels or ranges for a healthy individual), or may be specifically tailored for the person, if the person has a particular health condition that necessitates a target biomarker level to be different than that of the general population. Target biomarker levels may vary based on age, gender, weight, ethnicity, demographics. The biomarker index is representative of how much the measured biomarker profile (determined by measurements taken from the person, such as via a blood test) deviates from the target biomarker profile. The index may be a numeric index (such as a number between 1 and 100 or 0 and 1), an alphabetical index (such as a grade from F to A+), a color selected from a color gradient that represents the range of the index, a graphical icon that indicates progress towards the target profile, a combination of these, or any other visible or audible indicator that communicates a degree of deviation between the measured biomarker profile and the target biomarker profile. The form of the index may also be determined based on demographic features of the user, such as age and gender. For example, a child may be more responsive to an index that takes the form of a visual icon that changes as the child's measured biomarker profile approaches a target biomarker profile (*e.g.*, an animated dog that grows more active as the index increases or gold stars that increase in size and/or number as the index increases). An example of how the biomarker index may be computed is described in relation to FIG. 4.

In some implementations, rather than storing all of the above data in the health data database 106D, such data may be stored appropriately in any one or combination of the database 106A, 106B, and/or 106C. For example, the health data described above may be in a simplified form that includes sets of recommended nutrient templates for different demographics and medical treatments or conditions. In particular, a recommended nutrient template may have been identified as one that is most likely to improve a particular person's overall health, by most improving an efficacy of a medical treatment (as assessed by the biomarker index as described herein). Accordingly, the corresponding nutrient template may be recommended for food consumption to the particular person. In addition to storing the nutrient template itself, the nutrient template database 106A may also store the particular demographic features of the subset of people that exhibited the largest improvement. Furthermore, if enough studies similar to that described in FIG. 3 are performed, the nutrient templates may be stored in the database 106A with performance metrics (as measured by a biomarker index impact, as is described below, for example) for different demographic features. For example, a first nutrient template may perform well for young children, but not for adults. In this case, the relative change in a biomarker index for the subset of people described in relation to FIG. 3 may be stored, and may indicate a lower improvement in biomarker index for adults than for children.

As shown in FIG. 1, the database 106 includes the databases 106A-106D. Other databases may be included in the database 106, such as a foods database and/or a dietary programs database (as is described in U.S. Patent 9,011,153), a biomarker data database, a biomarker guidelines database, or any suitable combination thereof.

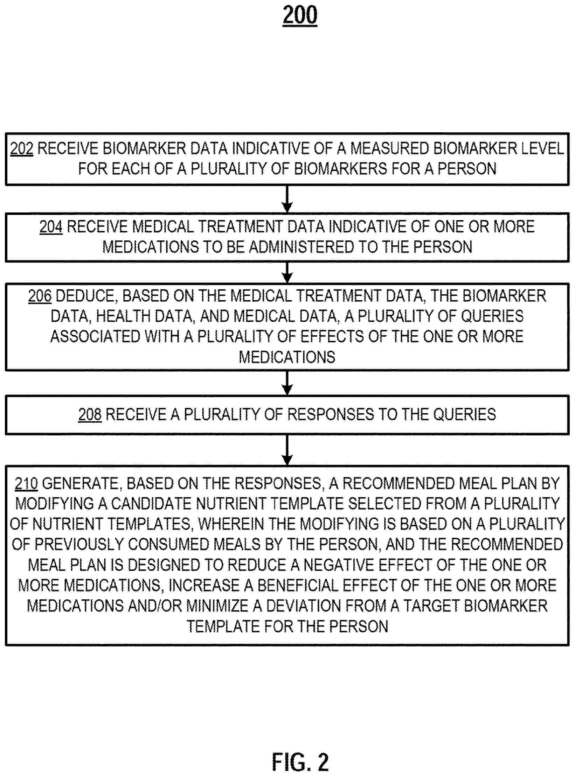

The server 104 receives input data over the user device 108 (such as biomarker data and medical treatment data associated with the user) from the person or a user authorized by the person, such as a clinician or dietician, and processes the input data with data stored in the database structure 106 to obtain food recommendations that, if consumed by the person, are predicted to improve the health of the person by improving the efficacy of a medical treatment to be administered to the person. In particular, the overall health of the person or the efficacy of the medical treatment may be predicted using a biomarker index that evaluates how closely the person's measured biomarker profile is aligned to the person's target biomarker profile. The process of providing a food recommendation is described in detail in relation to FIG. 2, and the process of evaluating a biomarker index is described in detail in relation to FIG. 4.

In some implementations, some or all of the components of the system 100 are part of a clinical testing setting, and the person interacting with the user interface 112 is a patient who is part of a clinical trial. In this case, some of the data provided over the user device 108 may conflict with other input data derived from clinical data obtained from a clinician. For example, as part of the clinical trial, the patient may not know the exact medication or dosage actually being administered, or the patient may be told that a different regimen is being administered than the regimen that is actually being administered. In this case, the data input over the user interface 112 may be overridden by the system 100 and replaced with the conflicting data derived from clinical data.

The components of the system 100 are shown in FIG. 1 as being distributed over multiple processing and storage devices connected via the network 102. Such an implementation may be appropriate for distributed computing over multiple communication systems including wireless and wired communication systems that share access to a common network resource. In some implementations, the system 100 is implemented in a cloud computing environment in which one or more of the components are provided by different processing and storage services connected via the Internet or other communications system. The arrangement and numbers of components shown in FIG. 1 are merely illustrative, and any suitable configuration may be used. The components of the system 100 of FIG. 1 may be arranged, distributed, and combined in any of a number of ways. For example, any of the databases within the database 106 may be stored on the server 104 and/or the user device 108. Furthermore, the processor 110 on the user device 108 may be configured to perform any or all of the functions of processors 105. The user device 108 may include an electronic database that is configured to store any or all of the data stored in database 106. Additionally, the functions performed by each of the components in the system 100 may be rearranged. In some implementations, the processor 110 may perform some or all of the functions of the processor 105 as described herein. For example, the processor 110 may compute the biomarker index, and/or may determine an appropriate food to recommend to the user. For ease of discussion, the remainder of this disclosure will often describe biomarker index determination and food recommendation techniques with reference to the system 100 of FIG. 1. However, any of the implementations described herein may be used, as well as any suitable variations of these systems.

FIG. 2 is a flowchart of a method 200 that may be implemented by the system 100 to improve the efficacy of a medical treatment to be administered to a person. The method 200 is described below as being performed by the processor 105, but the method 200 (or one or more steps of the method 200) may be instead performed by the processor 110 on the user device 108.

At step 202, the processor 105 receives biomarker data indicative of a measured biomarker level for each of a plurality of biomarkers for a person. As discussed above in relation to the database 106D, the biomarker data may include physiological measurements taken from a person, including without limitation blood test results, lipid panel test results, cholesterol screening results, psychological test results, fitness test results, cardiovascular assessments, side effects of a drug, or any other suitable physiological assessment that may be measured or acquired from a person. The processor 105 may further receive demographic information that describes the person, such as the person's age, weight, height, sex, ethnicity, body fat percentage, and/or medical condition.

At step 204, the processor 105 receives medical treatment data indicative of one or more medications to be administered to the person. As discussed above in relation to the database 106C, the medical treatment data may include information related to treatments for medical conditions that may be directly or indirectly related to a person's nutrition and includes medical conditions that are affected by the person's diet and/or that affect the person's diet. The medical treatment data may further include data that is descriptive of various parameters of the medical treatment, such as dosage, frequency, and duration of the medical treatment. At the time that the medical treatment data is received by the processor 105, the person may be considering undergoing the medical treatment, about to undergo the medical treatment, or already undergoing the medical treatment.

At step 206, the processor 105 deduces, based on the medical treatment data, the biomarker data, health data and medical data, a plurality of queries associated with a plurality of effects of the one or more medications. Each query may include a question of whether or not the person has observed an effect during the medical treatment, or the administration of the one or more medications. The effects may be determined to be common effects of the one or more medications and may include negative effects and/or positive effects. In particular, the negative effects may have undesirable consequences (e.g., bloating, gas, nausea, vomiting, diarrhea, tiredness, constipation, weight gain, weight loss, change in mood, or loss of hearing, vision, or appetite), while the positive effects may have desirable consequences (e.g., an intended effect of a medication, such as reducing cholesterol levels, reducing blood pressure, or weight loss).

In general, it may be undesirable to ask the person to evaluate whether or not all possible effects are experienced, as doing so would be time consuming and inefficient because not all effects are relevant to all medical treatments. Accordingly, the set of all possible negative and positive effects may be filtered to include only those associated with the one or more medications. The set of effects may include general effects, recorded effects, or both. To determine the general effects associated with the one or more medications, the processor 105 may cross reference the one or more medications from step 204 with the medical data stored in the database 106C to determine a set of general effects associated with the one or more medications. These may include side effects that are generally associated with the medication, as well as an intended effect of the medication. To determine the recorded effects associated with the one or more medications, the processor 105 may cross reference the one or more medications from step 204 and the biomarker data from step 202 with the health data stored in the database 106D to determine a set of common effects that were experienced by a subset of the population who were also administered the same one or more medications and had similar initial biomarker data as the person (as evaluated by a similar biomarker index, for example). The final set of queries deduced at step 206 may relate to the set of general effects alone, the set of recorded effects alone, effects in both the set of general effects and the set of recorded effects, or effects in either of the set of general effects or the set of recorded effects.

At step 208, the processor 105 receives a plurality of responses to the queries. In particular, each query may include a question of whether the person has experienced a particular effect, such as whether the person is bloated or nauseous. Moreover, the query may ask the person to quantitatively assess the severity of the effect, such as on a numeric scale of 1-10. When the query relates to an intended effect of the one or more medications, the response to the query may include measurable biomarker levels, such as cholesterol levels or blood pressure, or the person's weight.

At step 210, the processor 105 generates, based on the responses, a recommended meal plan by modifying a candidate nutrient template selected from a plurality of candidate nutrient templates, wherein the modifying is based on a plurality of previously consumed meals by the person, and the recommended meal plan is designed to reduce a negative effect of the one or more medications, increase a beneficial effect of the one or more medications and/or minimize a deviation from a target biomarker template for the person. In particular, the responses from step 208 are used to isolate a part of the population in the health data database 106D who experienced similar effects as the person. The processor 105 then obtains the biomarker data of this part of the population, and identified the particular segment of the part of the population that exhibited most improved biomarker indexes. The nutrient template assigned to that particular segment of the part of the population is then identified as a recommended nutrient template and is used for providing nutrition recommendations to the person. Accordingly, this recommended nutrient template is predicted to be the diet that would most help the person to improve his biomarker index, which is a metric that quantitatively evaluates the person's overall health or an efficacy of the medical treatment being used to treat the person. Details of how the biomarker index may be computed are described in relation to FIG. 4.

After the recommended nutrient template is identified, the overall target nutrient levels in the nutrient template are modified to take into account the person's previously consumed foods (such as the data stored in the nutritional data database 106B). Specifically, the target nutritional profile (including the overall target amounts of various nutrients for a person, for example) may be modified to subtract the person's consumed nutrient amounts (over the last time period, such as the last 12 hours, 24 hours, 2 days, 3 days, 4 day, 5 days, 6 days, or any other suitable time period, for example) to obtain an updated target nutritional profile. This updated target nutritional profile may then be used by the processor 105 to provide a food recommendation or a meal plan recommendation that would bring the person's consumed nutritional profile in better alignment with the target nutritional profile. Details of identifying food recommendations to align a person's diet with the person's dietary goals are described in detail in U.S. Patent 9,011,153.

In some implementations, an inference engine applies an artificial intelligence technique to deduce the plurality of queries at step 206, generate the recommended meal plan at step 210, or both. The artificial intelligence technique may use any number of machine learning and/or systems biology inference methods to determine common side effects from the medical treatment to be administered to the person, the person's biomarker data, the health data stored in the health data database 106D, the medical data stored in the medical data database 106C, or any subset thereof (to be included in the queries at step 206), or to generate the recommended meal plan at step 210. For example, the health data in the health database 106D may be used as a training set of data to extract particular features of a person's demographics, biomarker data, and/or diet and train a classifier that is capable of predicting side effects and/or the recommended meal plan.

FIG. 3 is a flowchart of a method 300 used by the processor 105 to identify a nutrient template that is predicted to perform best for a group of people (e.g., result in largest improvement in biomarker alignment). The method 300 may be used by the processor 105 to generate and store the health data in the health data database 106D. In general, the method 300 provides an analysis of the biomarker levels measured from a group of people and identifies an optimal nutrient template (e.g., set of target nutrient levels or ranges) that most improves an alignment between measured and target biomarker levels. The method 300 is described below as being performed by the processor 105, but the method 300 (or one or more steps of the method 300) may be instead performed by the processor 110 on the user device 108. An overview of the method 300 will first be provided, followed by illustrations of various implementations of the steps of the method 300.

As shown, the method 300 generally includes the steps of receiving a first input indicative of biomarker data acquired from a group of people before a time period (step 320), receiving multiple candidate nutrient templates (step 322), assigning a subset of the group of people to one of the candidate nutrient templates for nutrient consumption during the time period (step 324), and receiving a second input indicative of biomarker data acquired from the group of people after the time period (step 326). For each person in the group of people, the processor 105 assesses a first biomarker index representative of an alignment between the biomarker data in the first input and a recommended guideline, and a second biomarker index representative of an alignment between the biomarker data in the second input and the recommended guideline (step 328), and the processor 105 selects the nutrient template that is associated with the subset of the group of people that exhibited the largest improvement in alignment to the recommended guideline (step 330). By recommending a candidate nutrient template to each person in a subset of the group of people, the method 300 essentially reduces a variation in each subset's eating patterns. Doing so allows the method 300 to a determination of whether consumption of foods in compliance with a particular nutritional guideline (in the form of a candidate nutrient template, for example) improves a person's overall health or helps the person to achieve a specific health-related goal. Accordingly, the method 300 may be used to study a correlation between improvement in eating habits and an improvement in biomarker index.

The steps of the method 300 may be performed in any suitable order. For example, some steps of the method 300 may be performed simultaneously, in reverse order, or some steps may be omitted. For example, the step of receiving multiple candidate nutrient templates may occur before the first input is received. Moreover, rather than assessing the first biomarker index and the second biomarker index at step 328, the processor 105 may simply receive the first biomarker index and the second biomarker index from another source that performs the assessing. Although the biomarker index is generally described below in numeric terms for ease of illustration, it is understood that the index may one or more indicators of any suitable form capable of providing an assessment of the measured biomarker levels with respect to the recommended guidelines.

At step 320, the processor 105 receives a first input indicative of biomarker data acquired from a group of people before a time period. The biomarker data in the first input may be stored in the health data database 106D, and include a set of biomarker levels that are measured from each person in the group of people. At step 322, the processor 105 receives multiple candidate nutrient templates. The candidate nutrient templates may be stored in the nutrient templates database 106A, and include a set of nutrient target levels or a target nutritional profile. As described in relation to FIG. 1, the candidate nutrient templates may be specifically generated and/or modified for a particular person in a manner that is dependent on the person's health and demographic features.

At step 324, the processor 105 assigns a subset of the group of people to one of the candidate nutrient templates for nutrient consumption during the time period. In particular, the processor 105 may divide the group of people into multiple (e.g., K) subsets of people. This division may occur randomly to reduce a likelihood of introduction of bias in the subset selection. Alternatively, this division may occur in a manner that ensures a same or similar amount of variability of demographic data and/or similar amount of variability of nutritional indexes or eating habits in each subset. In some implementations, the group of people may all have similar demographic data, such as age range, gender, and medical conditions, for example. As an example, each person in the group of people may have a medical condition or a health problem that he may wish to address. One candidate nutrient template may be randomly assigned to each subset, such that each person in the group of people is instructed to consume food during the time period in accordance with the assigned candidate nutrient template. In a well-controlled study, the time period may have the same start and end times for each person in the group. Alternatively, the time period may have different start and end times for different people in the group, but the overall length of the time period for each person may be the same. In general, an important goal of assigning each subset to a candidate nutrient template (and later ensuring compliance with the assigned candidate nutrient template) is to reduce or remove a variability in the subset's eating habits and nutritional consumption, in order to determine whether setting specific nutritional guidelines (in the form of the candidate nutrient template) improves the subset's overall health.

The length of the time period may vary depending on the specific study that is being performed. For example, the time period may include a number of weeks or months. In some implementations, the method 300 of FIG. 3 is performed to assess an efficacy of a drug, and the time period may be set based on the drug. In an example, the biomarker data from the group of people may be actively monitored and recorded multiple times over the time period, such as once at 3 months, at 6 months, at 12 months, or any other suitable interval. In this case, at the beginning of the study, the length of time that it takes to see improvement in the group's biomarker indexes may be unknown. By actively monitoring and updating the biomarker data measurements multiple times, the study may reveal a suitable time period. In some implementations, the time period is set to a first time interval (such as 2 months, 4 months, or 6 months, for example). After the biomarker data is updated after the first time interval, the time period may be reset based on an amount of improvement exhibited by the biomarker data, or whether the updated biomarker data exhibits an improvement or not. For example, if the biomarker data exhibits no improvement, the time period may be reset to a second time interval that is longer than the first time interval (such as 4 months, 6, months, or 8 months, for example). Otherwise, the second time interval may be set to a shorter time window if the biomarker data exhibits some improvement, or an even shorter time window if the biomarker exhibits much improvement.

Instructing a person to consume food in accordance with an assigned candidate nutrient template may include providing food recommendations to the person, and may include any of the food recommendation or nutrition analysis programs as described in U.S. Patent 9,011,153. Specifically, the person's compliance with the candidate nutrient template may be tracked by evaluating a nutritional index that represents an alignment between the person's nutritional intake levels and target nutrient levels (as defined by the candidate nutrient template).

Each person reports the foods and/or nutrient amounts that the person has consumed during the time period, by entering food log entries into the user interface 112, for example. In an ideal case, each person in the group of people consumes food during the time period in compliance with his/her assigned candidate nutrient template, such that by the end of the time period each person's nutritional index indicates good or perfect alignment to the template. More realistically, however, the group of people will include a range of compliance to the templates. To identify a person in the group of people who has not complied with the assigned candidate nutrient template, the nutritional index may be compared to a threshold. If the person's index is below the threshold, that person may be removed from the group of people before proceeding with further steps of the method 300. Alternatively, that person's food consumption may be evaluated with respect to all other candidate nutrient templates. If the person's food consumption levels are in compliance with another candidate nutrient template (as measured by a nutritional index based on the other template), then the person may be reassigned to the corresponding subset. Furthermore, if enough people in the group of people do not comply with their respective candidate nutrient templates, but consumed foods in a similar manner as one another, a new subset may be formed within the group, with a new candidate nutrient template generated to conform with the subset's actual consumption levels.

At step 326, the processor 105 receives a second input indicative of biomarker data acquired from the group of people after the time period. Similar to the first input, the biomarker data in the second input may be stored in the health data database 106D, and include a set of biomarker levels that are measured from each person in the group of people after the group has consumed food in accordance with an assigned candidate nutrient template. As discussed above, persons within the group who have been identified to have not conformed with their assigned candidate nutrient template may be removed from the group, assigned to a different subset of people, or moved to a new subset of people. Accordingly, the subsets within the group of people are arranged in a way such that each subset represents people who have consumed food in a manner that is similar to one another (e.g., to reach the same target nutrient levels).

At step 328, the processor 105 assesses a first biomarker index representative of an alignment between the biomarker data in the first input and a recommended guideline and a second biomarker index representative of an alignment between the biomarker data in the second input and the recommended guideline. One implementation of assessing the first and second biomarker index is described in relation to FIG. 4. The first biomarker index may represent a baseline starting point assessment for each person's overall health, when the person may not be consuming food in accordance with any particular nutrient template. The second biomarker index may represent an assessment of the person's overall health, after the person has consumed food in compliance with a specific nutrient template. Accordingly, the difference between the first biomarker index and the second biomarker index may represent an improvement to the person's overall health that is attributable to the specific nutrient template. This improvement may be evaluated on an individual basis (the person's second biomarker index minus the person's first biomarker index), or on a subset basis (such as evaluating an aggregate difference between the set of second biomarker indexes for the subset minus the set of first biomarker indexes for the subset). When the improvement is evaluated on a subset basis, the aggregate difference may be computed by evaluating a statistic (such as a mean or a median) of the corresponding set of second biomarker indexes, evaluating a statistic (such as a mean or a median) of the corresponding set of first biomarker indexes, and taking the difference between the two statistics. The improvement in biomarker index may be referred to herein as a biomarker index impact, which is a quantitative measure of a predicted or measured change in biomarker index. This change may occur when a person changes his diet by consuming foods in accordance with a particular nutrient template.

At step 330, the processor 105 selects the nutrient template that is associated with the subset of the group of people that exhibited the largest improvement in alignment to the recommended guideline. As explained above, the improvement may be evaluated on an individual basis or on a subset basis. When evaluated on an individual basis, the improvement is represented by a set of individual differences, each representative of an improvement of a person's biomarker index in the corresponding subset. This set of individual differences may be processed before evaluating an aggregate difference that represents the overall improvement of the subset. For example, outliers in the set of individual differences may be discarded, and a statistic (that may include taking a mean, a median, and/or computing a normalization) of the remaining individual differences may be evaluated to represent the aggregate difference. In this case, the statistic may include a mean normalized by a standard deviation of the set of individual differences. Alternatively, when evaluated on a subset basis, the improvement may be represented as the aggregate difference described above.

The nutrient template that is selected at step 330 may be stored in the nutrient template database 106A, as described in relation to FIG. 1. Moreover, the predicted performance of the nutrient template in improvement a person's biomarker index (as indicated by the difference between the first and second biomarker indexes) may also be stored with the selected nutrient template, as well as any relevant demographic information pertaining to the group of people. When providing a nutrient template recommendation or a food recommendation to a person, the system 100 may evaluate the person's demographic information and identify a nutrient template that performed well for people with similar demographics. The other candidate nutrient templates that were not selected at step 330 and their predicted performances in improvement to a person's biomarker index may also be stored in the nutrient template database 106A, in case the selected nutrient template at step 330 is undesirable for recommendation (e.g., the person has a dietary restriction that precludes the person from consuming foods in accordance with the selected nutrient template). In this case, the nutrient template that resulted in the next largest improvement may be selected for recommendation to the person.

When all subsets have been considered, the subset within the group of people that exhibited the largest improvement is selected. In an example, when multiple subsets of people exhibit similarly large improvements, the subset with less variance in biomarker index difference may be chosen over the other subset with more variance. The corresponding nutrient template for the selected subset is identified, and may be provided to a user for recommendation for food consumption to improve the user's biomarker alignment to a recommended guideline. The user may be a person within the group of people described in the method 300, or may be a person outside the group of people.

In some implementations, certain steps of the method 300 of FIG. 3 are repeated multiple times. For example, steps 326 and 328 may be iteratively repeated while the group's biomarker data and food consumption data is updated and monitored. In this manner, the time course of any improvement may be determined. For example, the group's biomarker data and food consumption data may be updated on a weekly basis. A first subset within the group of people (consuming food in accordance with a first candidate nutrient template) may exhibit early improvement in biomarker index (e.g., during the first week) while a second subset within the group of people (consuming food in accordance with a second candidate nutrient template) may exhibit later improvement in biomarker index (e.g., during the second week). This result may cause a new study to be conducted, where the time course of the candidate nutrient templates is examined. In particular, a new time-varying candidate nutrient template may be generated to include the first candidate nutrient template during a first time period (e.g., the first week), and the second candidate nutrient template during a second time period (e.g., the second week), for example. In this way, the nutrient template that is ultimately recommended to a person may not be fixed for an amount of time. Instead, the recommended nutrient template may include different target nutrient levels at different times.

In some implementations, the method 300 of FIG. 3 is performed to assess an efficacy of a medical treatment, such as a drug. In particular, each person in the group of people may have the same or similar medical condition, and accordingly each person is administered or provided with a particular medical treatment, at least during the time period over which the person is instructed to consume food in accordance with the assigned candidate nutrient template. As is described in detail in relation to FIG. 5, the first and second biomarker indexes that are assessed at step 328 may include weights that indicate a high relevance of certain biomarkers that pertain to the medical treatment. For example, if administration of a drug is intended to lower a person's cholesterol levels, then the weight(s) that are used to apply to the deviation between the person's measured cholesterol levels and the person's target cholesterol levels may be set to higher values than weights for other biomarkers. In this manner, the improvement in biomarker index is a quantitative representation of an effectiveness of a medical treatment. When used with the method 300, an improvement in biomarker index can predict that a particular nutrient template is optimal for maximizing the efficacy of the treatment.

In an example, when the method 300 is used to assess a medical treatment's efficacy, the biomarker index may further include weights that indicate a high relevance of certain biomarkers that pertain to side effects associated with the medical treatment. For example, the medical treatment may be associated with nausea, drowsiness, skin reactions, pain, or constipation. While these side effects are often associated with qualitative descriptors, one way to quantify such side effects is to ask the persons to assess a severity of each side effect (on a numeric scale of 1 to 10, for example). Another way to quantify such side effects is on a binary scale, by asking the persons whether they are experiencing the side effect or not. In an example, a first set of one or more side effects are quantified according to severity (on a scale of 1 to 10, for example), while a second set of one or more side effects are quantified in a binary manner in assessing the same biomarker index (in a yes or no questionnaire, which may be represented as a 1 or a 2). In this case, a lower weight may be applied to the first set of side effects than the second set of side effects, to control for the higher scale that is used for the first set of side effects.

The biomarker index may take into account side effects of medical treatments by penalizing the index when severe side effects occur. For example, an n-th biomarker may include a side effect, such as nausea. The target biomarker level $g_n$ for nausea may be set to zero (on a scale of 1 to 10, for example), and the measured biomarker level $m_n$ for nausea may be set to the person's assessment of how much nausea he is experiencing (on a scale of 1 to 10, for example). The weight for nausea $w_n$ may reflect a relative importance of nausea with respect to other biomarkers (including other side effects, for example). Similar analysis may be performed for other side effects without departing from the scope of the present disclosure. By incorporating the efficacy and side effects of a medical treatment into the assessment of a biomarker index, the method 300 provides a way to recommend a specific diet that improves or optimizes the outcome of the treatment, or reduces an adverse effect of the treatment.

As explained above, the method 300 may be used to identify a correlation between improvement in eating habits and an improvement in biomarker index. In some implementations, a variation of the study described in method 300 may be performed to identify a correlation between a person's eating habits and a biomarker data. This variation may include versions of steps 326 and 328, where biomarker data is acquired from the group of people at step 326, as well as data related to the group's eating habits and dietary goals. The data related to the group's eating habits and dietary goals may be used to compute a nutritional index for each person, as described in Patent 9,011,153. The biomarker indexes assessed at step 328 are representative of each person's overall health or alignment to the person's health-related goals. The relationship between nutritional index and biomarker index may reveal a positive correlation, indicating that healthy eating habits may lead to good overall health.

FIG. 4 is a flowchart of a method 400 used by the processor 105 to assess a biomarker index, according to an illustrative implementation. The method 400 begins with receiving a call to the index function (step 420), identifying the user (step 422), and determining whether the index has been calculated since the last entry (decision block 424). If so, this means that no updated biomarker levels have been received since the last time the index was calculated, thereby precluding the need to update the index. In this case, the index is retrieved from memory (step 438) and returned before exiting (step 440). If the index has not been calculated since the last entry, the method 400 proceeds to steps 426, 428, 430, 434, and 436 to compute the biomarker index.

Computation of the biomarker index (and the use of measured biomarker levels, target biomarker levels, and biomarker weights) may be similar to the computation of a nutritional index (and the use of consumed nutrient levels, target nutrient levels, and nutrient weights), as is described in U.S. Patent 9,011,153. Specifically, the biomarker index may represent an aggregate alignment between the person's measured biomarker levels and target biomarker levels, and calculated as a weighted sum of deviations between the measurements and the targets.

The biomarker index is preferably a single marker that represents, in a cumulative fashion, how far the person's measured biomarker profile deviates from the target biomarker profile. In the examples described herein, the deviation for each biomarker is calculated as a difference between the target biomarker level (or range) and the measured biomarker level. However, any of several approaches for determining a deviation may be used. A deviation between a target (such as a desired level or range of a biomarker) and an attained amount (such as a measured level of a biomarker) provides an indication of an alignment between a goal (as may be defined by one or more targets) and an attainment (as may be defined by measured amounts of one or more biomarkers). Deviation can include a comparison between a specific target (which may or may not include a range) to a specific amount or level. A deviation can be determined for a specific component (e.g., a single biomarker) or for multiple components (e.g., multiple biomarkers compared on a biomarker-by-biomarker basis). Example suitable ways to determine a deviation include subtracting one value from another value to obtain a difference, computing a ratio between two values, any number of statistical approaches such as a standard deviation or statistical variance, pattern comparison and recognition, correlation approaches such as by comparing a curve or graph of one set of values to a corresponding curve or graph or another set of values, comparisons based on derived properties of the data sets, such as by regression-based or line-fitting approaches, or error estimation methods such as root-mean-squared. When multiple values of targets or attained levels are used, the data set may correspond to various biomarkers, time periods, individuals, groups of people, or any other suitable parameter. A profile of deviations may be obtained, such that the deviation profile is based on a comparison between a target biomarker profile for a person and a measured biomarker profile for the person. The deviation profile may include, for each biomarker in the target biomarker profile and/or the measured biomarker profile, an indication of whether the measured biomarker levels are in excess or deficit, relative to the target biomarker levels. An alignment can be determined based on one or more deviations to provide an indication of the person's overall health and compliance with recommended guidelines.

At step 426, the processor 105 retrieves or generates a user target vector TV, which is indicative of a target biomarker profile corresponding to target biomarker levels or ranges. The target biomarker profile may include data in accordance with a recommendation for the general population, or may be specifically tailored to the person, based on the person's demographic information, medical condition, medical treatment(s), and specific health-related goals. For example, a user-specific health program may be automatically generated based on the person's selected health-related goals and demographic features. The person's target profile includes target amounts (which may be fixed amounts or ranges) of various biomarkers.

The person may adjust these target amounts or ranges of biomarkers depending on the user's medical conditions (e.g., diabetes, heart disease) or susceptibility t certain medical conditions, specific demographic features (e.g., sex, age, race), a desire to achieve a particular physiological outcome (e.g., lose weight, gain weight, maintain weight, add strength, prevent cancer), a desired to conform to current medical thinking for specific demographic groups (e.g., female of child-bearing age, teenager, toddler, adult over 50), or any combination thereof.

Additionally or alternatively, a second user such as a clinician, analyst, researcher, or any trained individual may have access to view the person's inputs and may adjust the target amounts in the person's target biomarker profile. For example, the second user may adjust the user's target profile on a biomarker-by-biomarker basis. The second user may also adjust or select an appropriate target profile based on information in the original person's profile including the person's selected health-related goals and the person's demographic features. The second user may match the data in the person's profile with a health profile or may contact the person to set up a consultation. Furthermore, the second user may use the person's inputs and/or target profile to track clinical or other changes in the user's health profile.

The target biomarker level may be expressed as a function of the measured amount or range of amounts of another biomarker (e.g., the biomarkers for total cholesterol and HDL may be linked to each other, such as setting a target ratio of total cholesterol to HDL as 3.5 to 1, for example). References to target biomarker or biomarker level in this disclosure are understood to encompass targets expressed as fixed values, as ranges, or as functions of the goals of other biomarkers.

The target is determined for each biomarker in a set of target biomarkers. The target profile includes target biomarker levels, which may be generated in accordance with a general recommended guideline, or may be generated in a specific manner for an individual person. An example of a target profile is shown in column B of Table 1 below.

At step 428, the processor 105 retrieves or generates a measured biomarker profile corresponding to measured biomarker levels that are measured from the person and represented as a user measured vector MV. The measured biomarker profile includes the levels of the various biomarkers as measured from the person, on a biomarker-by-biomarker basis. For each biomarker in a set of biomarkers, the measured biomarker profile includes a measured biomarker level, which may include a quantity of the biomarker in the person's bloodstream, for example. An example of a measured biomarker profile is shown in column A of Table 1 below.

At step 430, the processor 105 retrieves or generates a weight profile, represented as a user weight vector WV. For a particular biomarker n, $w_n$ is a weight function (or simply, weight) applied to the deviation between the measured amount $m_n$ and the target biomarker level $g_n$. Different biomarkers may have different applied weights, depending on the person's specific health-related goals or medical condition. For example, a person with heart disease may be very affected by cholesterol levels, and thus a high cholesterol that exceeds a target level or range may be weighted much more heavily (to negative effect on the index) than a high amount of another biomarker. Moreover, when assessing the efficacy of a drug intended to reduce cholesterol levels, the weight for a biomarker related to cholesterol may be significantly larger than the weight for other biomarkers. Thus, for the same biomarker n, the weight associated with a biomarker for one person may be different from the weight associated with that biomarker for another person. In general, the magnitude of a weight for a biomarker may be indicative of a relative importance of that biomarker relative to other biomarkers for the medical treatment or medical condition related to the person. The weight function for a particular biomarker may be a constant value for all deviations from the target biomarker level, a piecewise-function that varies with the degree of deviation, a piecewise non-linear function, or a continuous linear or non-linear function. The weight function for the biomarker described herein may have similar characteristics as the weight function for a nutrient, described in U.S. Patent 9,011,153.

In an example, a recommended guideline may indicate that it is desirable for an amount of the biomarker HDL to be at least 60 mg/dL, no less than 40 mg/dL for men, and no less than 50 mg/dL for women. In this case, a target level for HDL may be set at 60 mg/dL for both men and women. A low weight $w_n$ (e.g., 0.1) may apply if the person has an HDL level that exceeds 60 mg/dL, to indicate a low negative impact on the person's biomarker index. The weight $w_n$ may be set to a higher value if the person has an HDL level that is below 60 mg/dL. In particular, the weight $w_n$ may depend on how much the measured HDL level is below 60 mg/dL, as well as the demographic information of the person (in one example, for men, $w_n$ is set to 0.9 if the HDL level is below 40 mg/dL, and 0.5 if the HDL level is between 40 to 60 mg/dL, and in another example, for women, $w_n$ is set to 0.9 if the HDL level is below 50 mg/dL, and 0.5 if the HDL level is between 50 to 60 mg/dL).

In an example, a recommended guideline may indicate that it is desirable for an amount of the biomarker LDL to be below 100 mg/dL. In this case, a target level for LDL may be set at 100 mg/dL. A low weight $w_n$ (e.g., 0.1) may apply if the person has an LDL level that is below 100 mg/dL, to indicate a low negative impact on the person's biomarker index. The weight $w_n$ may be set to a higher level (e.g., 0.9) if the person has an LDL level that is above 100 mg/dL, to indicate a large negative impact on the person's biomarker index. The $w_n$ may also be a function of how far above 100 mg/dL is the person's measured level of LDL.

At step 434, the processor 105 computes a product for each biomarker n by comparing the measured biomarker levels to the respective target biomarker levels on a biomarker-by-biomarker basis, and multiplying the corresponding weight $w_n$ by the deviation between the measured biomarker level $m_n$ and the target biomarker level $g_n$. In particular, the product computed at step 434 corresponds to a multiplication between the weight $w_n$ and a difference between $m_n$ and $g_n$. As is shown in FIG 4, the difference between $m_n$ and $g_n$ is represented as a difference in target vector TV and measured vector MV, or (TV-MV). In general, this difference may represent simply the difference between $g_n$ and $m_n$ ($g_n - m_n$, or $m_n - g_n$), or as the absolute value of the difference is used (as is shown in the numerator of Eq. 1 below), or as a normalized version of the absolute difference (as is shown in the numerator and denominator of Eq. 1 below). Moreover, the value for the corresponding weight $w_n$ may be dependent on whether the person exhibits an excess or a deficiency (e.g., the weight $w_n$ may depend on whether $m_n$ is greater than or less than $g_n$).

At step 436, the processor 105 sums the products computed at step 434 across the different biomarkers to obtain a biomarker index. The biomarker index is representative of an overall resemblance between the person's target biomarker profile and the person's measured biomarker profile and may be calculated in a manner similar to the nutritional index as described in U.S. Patent 9,011,153. For example, the biomarker index may be computed in accordance with the following set of three equations:

$$IC_n = w_n * \frac{\|m_n - g_n\|}{g_n} \tag{1}$$

$$IR = 1 - \frac{\sum_{n=1}^{N} IC_n}{\sum_{n=1}^{N} w_n} \tag{2}$$

$$I = \max(0, IR) \tag{3}$$

where

$IC_n$ is the index contribution of each biomarker *n;*
$w_n$ is a weight function or value applied to biomarker *n;*
$g_n$ is the target biomarker component level for biomarker *n;*
$m_n$ is a measured biomarker component level for biomarker *n.*
$N$ is the number of biomarkers in the set of biomarkers;
$IR$ is the raw index; and
$I$ is the index.

In this implementation, the index I corresponds to a number between zero and one. The closer the index is to "one" the closer the measured biomarker levels are to the person's target biomarker profile. Alternatively, an index near zero means that the measured biomarker levels are far from the target profile. Any suitable range (including a non-numeric range or a different numeric range) can be used for the index I, and the index I can take any form or value permitted by the range. The equations described herein are merely illustrative, and it will be understood that any suitable equation or function may be used (in combination with or in place of the equations described herein) for determining a value associated with an overall resemblance between the person's target profile and measured profile. After the index I is computed at step 436, the method 400 returns the index and exits (step 440).

Table 1 shows example measured levels $m_n$ of various biomarkers for a person (column A). Example personal target biomarker levels or ranges $g_n$, weights $a_n$ and $b_n$, active weights $w_n$, and index contribution $IC_n$ are also shown in Table 1. In the example shown in Table 1, when the measured biomarker level (column A) is equal to or within the range specified by the target biomarker level (column B), the active weight $w_n$ (column E) is set to zero, and the index contribution $IC_n$ of such biomarkers are set to zero. However, the active weights $w_n$ of such biomarkers may be set to another value (such as the larger value between the above weight $a_n$ and the below weight $b_n$) to reward the person for having a measured biomarker level equal to the target (or within the range specified by the target).

The difference between each measured biomarker level and the respective target biomarker level is computed to obtain an index contribution $IC_n$ (column F of Table 1). The index contribution $IC_n$ is computed in accordance with Eq. 1. When $g_n$ is specified as a target biomarker range, the value of $g_n$ is set to the upper boundary of the range when $m_n$ exceeds the target range, and the lower boundary of the range when $m_n$ is below the target range, though other ways of applying weights and using boundaries may be used, as is explained in detail below. In the example shown below in Table 1, the active weight $w_n$ is selected to be either an above weight $a_n$ (column C) or a below weight $b_n$ (column D). In general, the active weight $w_n$ may be selected based on any function, and may in particular be based on how far the measured biomarker level $m_n$ deviates from the target biomarker level or range $g_n$.

Assuming the twenty biomarkers shown in Table 1 are all N biomarkers, the corresponding index using Eq. 1-3 is 0.91. However, other biomarkers may also be considered and assigned appropriate target levels and weights. Examples of other biomarkers include other types of measurements that may be taken of a person to evaluate the person's overall health. For example, such measurements may include or be derived from physiological signals from the heart, lung, or another organ.

| Table 1 | A. Measured biomarker level $m_n$ | B. Personal target biomarker level or range $g_n$ | C. Above weight $a_n$ | D. Below weight $b_n$ | E. Active weight $w_n$ | F. Index Contribution $IC_n$ |
|---|---|---|---|---|---|---|
| ALT | 39 IU/L | 8-37 IU/L | 9 | 2 | 9 | 0.49 |
| Albumin | 4.5 g/dL | 3.9-5.0 g/dL | 8 | 8 | 0 | 0 |
| Alkaline Phosphatase | 156 IU/L | 44-147 IU/L | 8 | 2 | 8 | 0.49 |
| Blood Urea Nitrogen | 18 mg/dL | 10-20 mg/dL | 8 | 3 | 0 | 0 |
| Calcium | 9.5 mg/dL | 9.0-10.5 mg/dL | 9 | 3 | 0 | 0 |
| Chloride | 99 mEg/L | 98-106 mEg/L | 8 | 4 | 0 | 0 |
| Fasting Glucose | 82 mg/dL | 70-99 mg/dL | 7 | 6 | 0 | 0 |
| Phosphorous | 3.2 mg/dL | 2.4-4.1 mg/dL | 7 | 3 | 0 | 0 |
| Potassium | 3.2 mEg/L | 3.7-5.2 mEg/L | 5 | 5 | 5 | 0.68 |
| Sodium | 136 mEg/L | 135-145 mEg/L | 4 | 5 | 0 | 0 |
| Triglycerides | 120 mg/dL | 40-160 mg/dL | 4 | 6 | 0 | 0 |
| HDL | 80 mg/dL | >60 mg/dL | 0 | 6 | 0 | 0 |
| LDL | 105 mg/dL | <100 mg/dL | 8 | 0 | 8 | 0.4 |
| Total Cholesterol / HDL Ratio | 2.3 | <5.0 | 6 | 0 | 0 | 0 |
| WBC Leukocyte Count | 8900 cmm | 4300-10800 cmm | 9 | 9 | 0 | 0 |
| RBC Erythrocyte Count | 5.8 million cmm | 4.2-5.9 million cmm | 7 | 6 | 0 | 0 |
| Hematocrit | 51% | 45-52% | 5 | 5 | 0 | 0 |
| Hemoglobin | 15 g/dL | 13-18 g/dL | 4 | 8 | 0 | 0 |
| Platelet Count | 320000 mL | 150000-400000 mL | 7 | 7 | 0 | 0 |
| Vitamin D | 25 ng/mL | 30-74 ng/mL | 4 | 7 | 7 | 1.17 |

In an example, when $g_n$ is specified as a target biomarker range, the value of $g_n$ is set to the upper boundary of the range when $m_n$ exceeds the target range, and the lower boundary of the range when $m_n$ is below the target range, and only a single value for $w_n$ is used for each biomarker, depending on whether $m_n$ exceeds $g_n$ or vice versa. In another example, a range of values for $w_n$ may be used for a biomarker in computing an index contribution for the biomarker index. Specifically, an integral of the weight function may be computed. In an example, a target biomarker level $g_n$ is a single value (e.g., ci), and the weight function may include a step wise function, where $w_n$ is $w_{n1}$ (e.g., 6) when the measured biomarker level $m_n$ is between ci and $c_2$, and $w_{n2}$ (e.g., 10) when $m_n$ is above $c_2$, where $c_2 > c_1$ and $wn_2 > w_{n1}$. In this case, the higher weight value of $wn_2$ when $m_n$ is above $c_2$ represents a larger penalty on the user's biomarker index when the measured biomarker level is significantly above an ideal target biomarker level $c_1$, compared to the case when $m_n$ is between ci and $c_2$. When $m_n$ is between ci and $c_2$, the index contribution for the n-th biomarker (as represented above in Eq. 1) may be represented as

$$IC_n = w_{n1} * \frac{\|m_n - c_1\|}{c_1} \tag{4}$$

When $m_n$ is greater than $c_2$, the index contribution for the n-th biomarker may be represented as

$$IC_n = w_{n1} * \frac{\|c_2 - c_1\|}{c_1} + w_{n2} * \frac{\|m_n - c_2\|}{c_2} \tag{5}$$

or alternatively, as

$$IC_n = \frac{w_{n1} * \|c_2 - c_1\| + w_{n2} * \|m_n - c_2\|}{c_1 + c_2} \tag{6}$$

As indicated in Eqs. 5 and 6, the numerator in the index contribution for the n-th biomarker may be based at least in part on an integral of a weight function, or an area under the weight function. When the measured biomarker level is above the ideal target point (e.g., ci in the above example, which may be a lower boundary of a target range, an upper boundary of a target range, the target level itself, or a value within the target range, such as a midpoint), the limits of the integral may range from an ideal target point to the measured biomarker level. Alternatively, when the measured biomarker level is below the ideal target point, the limits of the integral may range from the measured biomarker level to the ideal target point. In either case, the integral may be normalized in a piecewise manner, where each section of the weight function is normalized by the corresponding boundary (such as is shown in Eq. 5) or in a non-piecewise manner, where the numerator represents the entire integral, and is normalized by a function of the corresponding boundaries (such as the sum of ci and $c_2$, which is shown in Eq. 6).

In general, the biomarker index may be used to minimize a deviation between the measured biomarker profile for the person and the target biomarker template for the person. In this case, the health data stored in the health data database 106D is mined to identify a group of people having similar characteristics as the person, such as having similar demographics and who underwent the same medical treatment as the person. The time course of biomarker indexes (as described above) for the group of people is examined. Those within the group who exhibited most improved biomarker indexes or the highest biomarker indexes at the end of treatment (or during an intermediate time during treatment) may be examined further to determine their diets, and a similar diet or nutrient template may be recommended to the person, as a diet that is predicted to have the largest predicted impact on the biomarker index (i.e., the biomarker index impact) that would best align the person's biomarker measurements with target levels.

In one example, the biomarker index is used to improve or maximize an efficacy of a medical treatment. In this case, the weights for biomarkers that have to do with the intended effect of the medical treatment may be set to high values, while the weights for other biomarkers may be set to zero or relatively low values. Accordingly, when the biomarker indexes for the population of people in the health data (stored in the health data database 106D) are assessed, those who exhibited the largest improvement in biomarker index (or the overall highest biomarker indexes at the end of treatment) correspond to the people who had maximal efficacy of the medical treatment, when compared to other people who were undergoing the same medical treatment but ate different diets.

In one example, the biomarker index is used to reduce or minimize a negative effect of a medical treatment. In this case, the weights for biomarkers that have to do with adverse unintentional consequences of the medical treatment may be set to high values, while the weights for other biomarkers may be set to zero or relatively low values. In this case, a biomarker may correspond to such adverse side effects, and may be evaluated based on patient responses to a questionnaire as binary responses or on a scale, such as 0-10 (where the target biomarker level corresponds to zero).

Accordingly, when the biomarker indexes for the population of people in the health data are assessed, those who exhibited the largest improvement in biomarker index (or the overall highest biomarker indexes at the end of treatment) correspond to the people who experienced the least amount of adverse side effects of the medical treatment.

In some implementations, the biomarker index is used to both improve an efficacy of a medical treatment while reducing a negative effect of the medical treatment. In this case, the weights for biomarkers that have to do with the intended effect of the medical treatment, as well as weights that have to do with adverse unintentional consequences of the medical treatment may be set to high values, while the weights for other biomarkers may be set to zero or relatively low values. Accordingly, when the biomarker indexes for the population of people in the health data are assessed, those who exhibited the largest improvement in biomarker index (or the overall highest biomarker indexes at the end of treatment) correspond to the people who simultaneously experienced high efficacy of the medical treatment and low adverse side effects of the medical treatment.

In an example, a goal of the drug may be to lower a person's LDL level. In this case, the weight $w_n$ applied to the LDL biomarker may be set to a high value relative to the weights of other biomarkers, to reflect the importance of the LDL biomarker relative to other biomarkers. In an example, the drug may be administered or provided to each person in the group of people described in relation to FIG. 4. Each person's response to the drug may be monitored by measuring the person's biomarker levels (especially the person's LDL level) before and after the drug is administered, as well as optionally recording each person's side effects in reaction to the drug, if any. The subset of the group of people who had the best response and reaction to the drug (as measured by a largest improvement in biomarker index, for example) is selected, and the corresponding nutrient template is identified for recommendation.

Continuing with this example of using the biomarker index to evaluate drug efficacy, the weight $w_n$ for LDL may be set to a high value if the person's LDL level is within the target range. For example, an optimal LDL level may be below 100 mg/dL, a near optimal LDL level may be between 100 to 129 mg/dL, a borderline high LDL level may be between 130 to 159 mg/dL, a high LDL level may be between 160 to 189 mg/dL, and a very high LDL level may be above 189 mg/dL. The weight $w_n$ may be set to increasing values as the LDL level increases. In this manner, the resulting biomarker index includes a large index contribution component from LDL, and is increasingly penalized as the LDL level increases further above the target level or range.

When evaluating drug efficacy, one or more side effects of the drug may be factored into the calculation of the biomarker index. For example, the above-described group of people may be monitored for any side effects in reaction to the drug, such as nausea, fever, headache, drowsiness, skin irritation, discomfort, clear skin, increased energy, or any other side effect. Each person may individually report a severity of how much he is experiencing each side effect (on a scale of 0 to 10, for example), and the side effects may be treated as biomarkers, with target biomarker levels $g_n$ (e.g., 0 for a negative side effect such as nausea or fever, or 10 for a positive side effect such as clear skin or increased energy), measured biomarker levels $m_n$ (e.g., as reported by the individuals), and weights $w_n$. Such side effects may be quantified by methods described in relation to FIG. 3.

As described herein, a biomarker index is a way of assessing and monitoring a person's overall health, and may be used to assess changes to the person's overall health as his diet changes with time. As discussed above, the biomarker index is representative of how much the measured biomarker profile deviates from the target biomarker profile. The index may be a numeric index (such as a number between 1 and 100 or 0 and 1), an alphabetical index (such as a grade from F to A+), a color selected from a color gradient that represents the range of the index, a graphical icon that indicates progress towards the target profile, a combination of these, or any other visible or audible indicator that communicates a degree of deviation between the measured biomarker profile and the target biomarker profile. In some implementations, the biomarker index is a single indicator, such as a single aggregate number that is representative of an aggregate alignment of multiple biomarkers simultaneously. For example, after an optimal nutrient template is identified (using the method 300 described in relation to FIG. 3, for example) for a specific person, foods may be recommended to that person in accordance with the optimal nutrient template. Example food and meal recommendation systems and methods are described in detail in U.S. Patent 9,011,153, any of which may be used in accordance with the recommendation systems described herein.

The systems and methods disclosed herein may be provided for one biomarker or for multiple biomarkers. However, the systems and methods are particularly advantageous when applied to multiple biomarkers (e.g., to three or more biomarkers) to simultaneously assess a health status for one or more persons. Assessing the overall health of a person or recommending foods when multiple biomarkers are at stake can be a particularly challenging computational problem. This difficulty is due in part to the large number of measurements and evaluations that should be considered when evaluating a person's overall health, as well as the staggering number of available foods (currently estimated as a few tens of thousands), the variability in nutritional value of these foods (both in terms or amount and types of nutrients), varying portion sizes for the same food, and user-specific factors that must be considered in order to provide useful assessments and recommendations.

The steps of the methods 200, 300, and 400 are in the shown order for illustrative purposes only, and one of ordinary skill in the art will understand that any step may be omitted, the order of the steps may be modified, or any steps may be performed simultaneously, without departing from the disclosure.

FIG. 5 is a block diagram of a computing device, such as any of the components of the system of FIG. 1, for performing any of the processes described herein. Each of the components of these systems may be implemented on one or more computing devices 500. In certain aspects, a plurality of the components of these systems may be included within one computing device 500. In certain implementations, a component and a storage device may be implemented across several computing devices 500.

The computing device 500 includes at least one communications interface unit, an input/output controller 510, system memory, and one or more data storage devices. The system memory includes at least one random access memory (RAM 502) and at least one read-only memory (ROM 504). All of these elements are in communication with a central processing unit (CPU 506) to facilitate the operation of the computing device 500. The computing device 500 may be configured in many different ways. For example, the computing device 500 may be a conventional standalone computer or alternatively, the functions of computing device 500 may be distributed across multiple computer systems and architectures. In FIG. 5, the computing device 500 is linked, via network or local network, to other servers or systems.

The computing device 500 may be configured in a distributed architecture, wherein databases and processors are housed in separate units or locations. Some units perform primary processing functions and contain at a minimum a general controller or a processor and a system memory. In distributed architecture implementations, each of these units may be attached via the communications interface unit 508 to a communications hub or port (not shown) that serves as a primary communication link with other servers, client or user computers and other related devices. The communications hub or port may have minimal processing capability itself, serving primarily as a communications router. A variety of communications protocols may be part of the system, including, but not limited to: Ethernet, SAP, SAS™, ATP, BLUETOOTH™, GSM and TCP/IP.

The CPU 506 includes a processor, such as one or more conventional microprocessors and one or more supplementary co-processors such as math co-processors for offloading workload from the CPU 506. The CPU 506 is in communication with the communications interface unit 508 and the input/output controller 510, through which the CPU 506 communicates with other devices such as other servers, user terminals, or devices. The communications interface unit 508 and the input/output controller 510 may include multiple communication channels for simultaneous communication with, for example, other processors, servers or client terminals.

The CPU 506 is also in communication with the data storage device. The data storage device may include an appropriate combination of magnetic, optical or semiconductor memory, and may include, for example, RAM 502, ROM 504, flash drive, an optical disc such as a compact disc or a hard disk or drive. The CPU 506 and the data storage device each may be, for example, located entirely within a single computer or other computing device; or connected to each other by a communication medium, such as a USB port, serial port cable, a coaxial cable, an Ethernet cable, a telephone line, a radio frequency transceiver or other similar wireless or wired medium or combination of the foregoing. For example, the CPU 506 may be connected to the data storage device via the communications interface unit 508. The CPU 506 may be configured to perform one or more particular processing functions.

The data storage device may store, for example, (i) an operating system 512 for the computing device 500; (ii) one or more applications 514 (e.g., computer program code or a computer program product) adapted to direct the CPU 506 in accordance with the systems and methods described here, and particularly in accordance with the processes described in detail with regard to the CPU 506; or (iii) database(s) 516 adapted to store information that may be utilized to store information required by the program.

The operating system 512 and applications 514 may be stored, for example, in a compressed, an uncompiled and an encrypted format, and may include computer program code. The instructions of the program may be read into a main memory of the processor from a computer-readable medium other than the data storage device, such as from the ROM 504 or from the RAM 502. While execution of sequences of instructions in the program causes the CPU 506 to perform the process steps described herein, hard-wired circuitry may be used in place of, or in combination with, software instructions for implementation of the processes of the present disclosure. Thus, the systems and methods described are not limited to any specific combination of hardware and software.

Suitable computer program code may be provided for performing one or more functions in relation to improving biomarker alignment as described herein. The program also may include program elements such as an operating system 512, a database management system and "device drivers" that allow the processor to interface with computer peripheral devices (*e.g.*, a video display, a keyboard, a computer mouse, *etc.)* via the input/output controller 510.

The term "computer-readable medium" as used herein refers to any non-transitory medium that provides or participates in providing instructions to the processor of the computing device 500 (or any other processor of a device described herein) for execution. Such a medium may take many forms, including but not limited to, non-volatile media and volatile media. Non-volatile media include, for example, optical, magnetic, or opto-magnetic disks, or integrated circuit memory, such as flash memory. Volatile media include dynamic random access memory (DRAM), which typically constitutes the main memory. Common forms of computer-readable media include, for example, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM or EEPROM (electronically erasable programmable read-only memory), a FLASH-EEPROM, any other memory chip or cartridge, or any other non-transitory medium from which a computer can read.

Various forms of computer readable media may be involved in carrying one or more sequences of one or more instructions to the CPU 506 (or any other processor of a device described herein) for execution. For example, the instructions may initially be borne on a magnetic disk of a remote computer (not shown). The remote computer can load the instructions into its dynamic memory and send the instructions over an Ethernet connection, cable line, or even telephone line using a modem. A communications device local to a computing device 500 (*e.g.*, a server) can receive the data on the respective communications line and place the data on a system bus for the processor. The system bus carries the data to main memory, from which the processor retrieves and executes the instructions. The instructions received by main memory may optionally be stored in memory either before or after execution by the processor. In addition, instructions may be received via a communication port as electrical, electromagnetic or optical signals, which are exemplary forms of wireless communications or data streams that carry various types of information.

**Claims**

1. A system for providing a personalized meal plan for improving an efficacy of a medical treatment for a person, the system comprising:

   one or more data storage devices for storing one or more electronic knowledge bases comprising (i) health data collected from a plurality of data sources and related to a plurality of demographics, (ii) medical data for a plurality of medical treatments, and (iii) nutritional data including a plurality of meals previously consumed by the person and a library of nutrient contents for a plurality of nutrient templates; an inference engine implemented on one or more processors including hardware and software, the inference engine being configured to:

   (a) receive biomarker data indicative of a measured biomarker level for each of a plurality of biomarkers for the person,
   (b) receive medical treatment data indicative of one or more medications to be administered to the person,
   (c) deduce, using an artificial intelligence technique using machine learning, based on the medical treatment data, the biomarker data, the health data, and the medical data from the knowledge base, a plurality of queries associated with a plurality of effects of the one or more medications, the queries comprising a question of whether or not the person has observed an effect during the medical treatment or the administration of the one or more medications,
   (d1) receive a plurality of inputs indicative of responses to the queries,
   (d2) based on the plurality of responses to the queries, isolate in the one or more electronic knowledge bases a data set indicative of a part of the population who experienced similar effects as the person, and identify a segment of the data set indicative of a part of the population that exhibited most improved biomarker indexes, and
   (e) generate by the one or more processors, based on the inputs, a recommended meal plan by assigning as a candidate nutrient template the nutrient template assigned to the segment of the part of the population that exhibited most improved biomarker indexes, and modifying the candidate nutrient template, wherein the modifying is based on a subset of the previously consumed meals and comprises subtracting nutrient amounts consumed by the person over a time period prior to generation of the meal plan to obtain an updated target nutritional profile, and the recommended meal plan is designed to reduce a negative effect of the one or more medications, increase a beneficial effect of the one or more medications and/or minimize a deviation from a target biomarker template for the person;
   f) generate, based on the recommended meal plan, a predicted biomarker index indicative of an aggregate alignment between the target biomarker template and the biomarker data for the person upon following the recommended meal plan for a predetermined time period, and
   a communications port for providing the recommended meal plan to a user interface and display the meal plan and the predicted biomarker index to the person for selection by the person.

2. The system of any of the foregoing claims, wherein the inference engine is further configured to (g) generate, based on the biomarker data, a biomarker index indicative of an aggregate alignment between the target biomarker template and the biomarker data for the person, and the communications port is further for providing the biomarker index to the person.

3. The system of claim 2, wherein the biomarker index is based on a weighted function of deviations between target biomarker levels defined by the target biomarker template and measured biomarker levels in the biomarker data for the person.

4. The system of claim 3, wherein the weighted function includes a biomarker coefficient applied to the deviation for each respective target biomarker, the biomarker coefficient being indicative of a relative importance of the respective target biomarker to other biomarkers in the target biomarker template for the one or more medications.

5. The system of any of the foregoing claims, wherein the health data includes biomarker data for a plurality of persons who were previously administered the one or more medications and the corresponding nutrients consumed by the plurality of persons during the period of time corresponding to the administration of the one or more medications.

6. The system of any of the foregoing claims, wherein the plurality of queries corresponds to common side effects associated with the one or more medications.

7. The system of any of the foregoing claims, wherein the negative effect corresponds to one or more undesirable side effects that negatively impact efficacy of the one or more medications or negatively impacts compliance with a prescribed regimen of the one or more medications.

8. The system of claim 7, wherein the one or more undesirable side effects include at least one of bloating, gas, nausea, vomiting, diarrhea, tiredness, constipation, weight gain, weight loss, and any combination of two or more of the foregoing.

9. The system of any of the foregoing claims, wherein the beneficial effect corresponds to one or more target therapeutic effects of the one or more medications.

10. The system of any of the foregoing claims, wherein the target biomarker template is determined based on consensus guidelines regarding target biomarker ranges for a healthy individual, the person's demographic information, and the medical treatment.

11. The system of claim 10, wherein the demographic information includes genetic information obtained based on a biological sample of the person.

12. The system of any of the foregoing claims, wherein the one or more electronic knowledge bases further comprise (iv) clinical data related to a clinical testing setting, wherein the inference engine is further configured to override an input from the person with respect to the one or more medications if the input conflicts with one or more inputs derived from the clinical data.

13. The system of any of the foregoing claims, further comprising a user interface for a clinician associated with the person, the user interface including a dashboard having options to display information related to the person, including the person's demographic information, the health data related to the person's demographics, the measured biomarker levels for the person, the one or more medications to be administered to the person, amounts of nutrients in the plurality of meals previously consumed by the person, and the recommended meal plan.

14. The system of any of the foregoing claims, further comprising a user interface for the person, the user interface including options to display information related to the person, including the person's demographic information, the measured biomarker levels for the person, the one or more medications to be administered to the person, amounts of nutrients in the plurality of meals previously consumed by the person, and the recommended meal plan.

**Patentansprüche**

1. System zum Bereitstellen eines personalisierten Speiseplans zum Verbessern einer Wirksamkeit einer medizinischen Behandlung für eine Person, wobei das System Folgendes umfasst:
eine oder mehrere Datenspeichervorrichtungen zum Speichern einer oder mehrerer elektronischen Wissensbasen, umfassend (i) Gesundheitsdaten, die aus einer Vielzahl von Datenquellen gesammelt werden und eine Vielzahl von demographischen Daten betreffen, (ii) medizinische Daten für eine Vielzahl von medizinischen Behandlungen und (iii) Ernährungsdaten, die eine Vielzahl von Mahlzeiten, die zuvor durch die Person konsumiert wurden, und eine Bibliothek von Nährstoffinhalten für eine Vielzahl von Nährstoffmustern beinhalten; eine Inferenz-Engine, die auf einem oder mehreren Prozessoren implementiert ist und Hardware und Software beinhaltet, wobei die Inferenz-Engine zu Folgendem konfiguriert ist:

(a) Empfangen von Biomarkerdaten, die einen gemessenen Biomarkerspiegel für jeden aus einer Vielzahl von Biomarkern für die Person angeben,

(b) Empfangen von Daten zur medizinischen Behandlung, die ein oder mehrere der Person zu verabreichende Arzneimittel angeben,

(c) unter Verwendung einer Technik der künstlichen Intelligenz unter Verwendung von maschinellem Lernen auf Grundlage der Daten zur medizinischen Behandlung, der Biomarkerdaten, der Gesundheitsdaten und der medizinischen Daten aus der Wissensbasis, Herleiten einer Vielzahl von Anfragen, die einer Vielzahl von Wirkungen des einen oder der mehreren Arzneimittel zugeordnet ist, wobei die Anfragen eine Frage umfassen, ob die Person eine Wirkung während der medizinischen Behandlung oder der Verabreichung des einen oder der mehreren Arzneimittel beobachtet hat oder nicht,

(d1) Empfangen einer Vielzahl von Eingaben, die Antworten auf die Anfragen angeben,

(d2) auf Grundlage der Vielzahl von Antworten auf die Anfragen, Isolieren eines Datensatzes in der einen oder den mehreren elektronischen Wissensbasen, der einen Teil der Bevölkerung angibt, der ähnliche Wirkungen wie die Person erfahren hat, und Identifizieren eines Segments des Datensatzes, das einen Teil der Bevölkerung angibt, der die am meisten verbesserten Biomarkerindizes aufwies, und

(e) auf Grundlage der Eingaben, Generieren eines empfohlenen Speiseplans durch den einen oder die mehreren Prozessoren durch Zuweisen des Nährstoffmusters, das dem Segment des Teils der Bevölkerung, der die am meisten verbesserten Biomarkerindizes aufwies, als mögliches Nährstoffmuster, und Modifizieren des möglichen Nährstoffmusters, wobei das Modifizieren auf einer Teilmenge der zuvor konsumierten Mahlzeiten basiert und Subtrahieren der durch die Person über einen Zeitraum vor der Generierung des Speiseplans konsumierten Nährstoffmengen umfasst, um ein aktualisiertes Ziel-Ernährungsprofil zu erlangen, und der empfohlene Speiseplan dazu ausgelegt ist, eine negative Wirkung des einen oder der mehreren Arzneimittel zu reduzieren, eine vorteilhafte Wirkung des einen oder der mehreren Arzneimittel zu erhöhen und/oder eine Abweichung von einem Ziel-Biomarkermuster für die Person zu minimieren;

f) auf Grundlage des empfohlenen Speiseplans, Generieren eines vorhergesagten Biomarkerindexes, der eine aggregierte Angleichung zwischen dem Ziel-Biomarkermuster und den Biomarkerdaten für die Person angibt, wenn die Person den empfohlenen Speiseplan für einen vorbestimmten Zeitraum befolgt, und

einen Kommunikationsport zum Bereitstellen des empfohlenen Speiseplans an eine Benutzerschnittstelle und Anzeigen des Speiseplans und des vorhergesagten Biomarkerindexes für die Person zur Auswahl durch die Person.

2. System nach einem der vorhergehenden Ansprüche, wobei die Inferenz-Engine ferner dazu konfiguriert ist, (g) auf Grundlage der Biomarkerdaten einen Biomarkerindex zu generieren, der eine aggregierte Angleichung zwischen dem Ziel-Biomarkermuster und den Biomarkerdaten für die Person angibt, und der Kommunikationsport ferner zum Bereitstellen des Biomarkerindexes für die Person dient.

3. System nach Anspruch 2, wobei der Biomarkerindex auf einer gewichteten Funktion von Abweichungen zwischen den durch das Ziel-Biomarkermuster definierten Ziel-Biomarkerspiegeln und den gemessenen Biomarkerspiegeln in den Biomarkerdaten für die Person basiert.

4. System nach Anspruch 3, wobei die gewichtete Funktion einen Biomarkerkoeffizienten beinhaltet, der auf die Abweichung für jeden jeweiligen Ziel-Biomarker angewendet wird, wobei der Biomarkerkoeffizient eine relative Bedeutung des jeweiligen Ziel-Biomarkers für andere Biomarker in dem Ziel-Biomarkermuster für das eine oder die mehreren Arzneimittel angibt.

5. System nach einem der vorhergehenden Ansprüche, wobei die Gesundheitsdaten Biomarkerdaten für eine Vielzahl von Personen, der zuvor das eine oder die mehreren Arzneimittel verabreicht wurde, und die entsprechenden Nährstoffe, die durch die Vielzahl von Personen während des Zeitraums konsumiert wurden, der der Verabreichung des einen oder der mehreren Arzneimittel entspricht, beinhalten.

6. System nach einem der vorhergehenden Ansprüche, wobei die Vielzahl von Anfragen den häufigen Nebenwirkungen entspricht, die dem einen oder den mehreren Arzneimitteln zugeordnet sind.

7. System nach einem der vorhergehenden Ansprüche, wobei die negative Wirkung einer oder mehreren unerwünschten Nebenwirkungen entspricht, die die Wirksamkeit des einen oder der mehreren Arzneimittel negativ beeinflussen oder die Einhaltung eines verordneten Regimes des einen oder der mehreren Arzneimittel negativ beeinflussen.

8. System nach Anspruch 7, wobei die eine oder mehreren unerwünschten Nebenwirkungen mindestens eines von Völlegefühl, Blähungen, Übelkeit, Erbrechen, Durchfall, Müdigkeit, Verstopfung, Gewichtszunahme, Gewichtsverlust und eine beliebige Kombination von zwei oder mehr der vorgenannten beinhalten.

9. System nach einem der vorhergehenden Ansprüche, wobei die vorteilhafte Wirkung einer oder mehreren therapeutischen Zielwirkungen des einen oder der mehreren Arzneimittel entspricht.

10. System nach einem der vorhergehenden Ansprüche, wobei das Ziel-Biomarkermuster auf Grundlage von Konsensrichtlinien betreffend Ziel-Biomarkerbereiche für ein gesundes Individuum, den demografischen Informationen der Person und der medizinischen Behandlung bestimmt ist.

11. System nach Anspruch 10, wobei die demographische Informationen genetische Informationen beinhalten, die auf Grundlage einer biologischen Probe der Person erlangt werden.

12. System nach einem der vorhergehenden Ansprüche, wobei die eine oder mehreren elektronischen Wissensbasen ferner (iv) klinische Daten umfassen, die eine klinische Testumgebung betreffen, wobei die Inferenz-Engine ferner dazu konfiguriert ist, eine Eingabe von der Person in Bezug auf das eine oder die mehreren Arzneimittel zu überschreiben, wenn die Eingabe mit einer oder mehreren Eingaben kollidiert, die aus den klinischen Daten abgeleitet sind.

13. System nach einem der vorhergehenden Ansprüche, ferner umfassend eine Benutzerschnittstelle für einen Arzt, der der Person zugeordnet ist, wobei die Benutzerschnittstelle ein Dashboard beinhaltet, das Optionen zum Anzeigen von Informationen betreffend die Person aufweist, einschließlich der demografischen Informationen der Person, der Gesundheitsdaten betreffend die demographischen Daten der Person, der gemessenen Biomarkerspiegel für die Person, des einen oder der mehreren Arzneimittel, die der Person zu verabreichen sind, Mengen an Nährstoffen in der Vielzahl von Mahlzeiten, die zuvor durch die Person konsumiert wurde, und des empfohlenen Speiseplans.

14. System nach einem der vorhergehenden Ansprüche, ferner umfassend eine Benutzerschnittstelle für die Person, wobei die Benutzerschnittstelle Optionen zum Anzeigen von Informationen betreffend die Person aufweist, einschließlich der demografischen Informationen der Person, der gemessenen Biomarkerspiegel für die Person, des einen oder der mehreren Arzneimittel, die der Person zu verabreichen sind, Mengen an Nährstoffen in der Vielzahl von Mahlzeiten, die zuvor durch die Person konsumiert wurde, und des empfohlenen Speiseplans.

**Revendications**

1. Système permettant de fournir un plan de repas personnalisé pour améliorer l'efficacité d'un traitement médical pour une personne, le système comprenant :
   un ou plusieurs dispositifs de stockage de données pour stocker une ou plusieurs bases de connaissances électroniques comprenant (i) des données de santé collectées à partir d'une pluralité de sources de données et liées à une pluralité de données démographiques, (ii) des données médicales pour une pluralité de traitements médicaux, et (iii) des données nutritionnelles comportant une pluralité de repas consommés précédemment par la personne et une bibliothèque de teneurs en nutriments pour une pluralité de modèles de nutriments ; un moteur d'inférence mis en œuvre sur un ou plusieurs processeurs comportant du matériel et des logiciels, le moteur d'inférence étant configuré pour :

   (a) recevoir des données de biomarqueurs indiquant un niveau de biomarqueur mesuré pour chacun d'une pluralité de biomarqueurs pour la personne,
   (b) recevoir des données de traitement médical indiquant un ou plusieurs médicaments à administrer à la personne,
   (c) déduire, à l'aide d'une technique d'intelligence artificielle utilisant l'apprentissage automatique, sur la base des données de traitement médical, des données de biomarqueurs, des données de santé et des données médicales de la base de connaissances, une pluralité de requêtes associées à une pluralité d'effets des un ou plusieurs médicaments, les requêtes comprenant une question de savoir si la personne a observé ou non un effet au cours du traitement médical ou de l'administration des un ou plusieurs médicaments,
   (d1) recevoir une pluralité d'entrées indiquant des réponses aux requêtes,
   (d2) sur la base de la pluralité de réponses aux requêtes, isoler dans les une ou plusieurs bases de connaissances électroniques un ensemble de données indiquant une partie de la population qui a subi des effets similaires à ceux de la personne, et identifier un segment de l'ensemble de données indiquant une partie de la population qui a présenté les indices de biomarqueurs les plus améliorés, et

(e) générer par les un ou plusieurs processeurs, sur la base des entrées, un plan de repas recommandé en attribuant comme modèle de nutriment candidat le modèle de nutriment attribué au segment de la partie de la population qui a présenté les indices de biomarqueurs les plus améliorés, et en modifiant le modèle de nutriment candidat, dans lequel la modification est basée sur un sous-ensemble des repas consommés précédemment et comprend la soustraction des quantités de nutriments consommées par la personne pendant une période avant la génération du plan de repas pour obtenir un profil nutritionnel cible mis à jour, et le plan de repas recommandé est conçu pour réduire un effet négatif des un ou plusieurs médicaments, augmenter un effet bénéfique des un ou plusieurs médicaments et/ou minimiser un écart par rapport à un modèle de biomarqueur cible pour la personne ;

f) générer, sur la base du plan de repas recommandé, un indice de biomarqueur prédit indiquant un alignement global entre le modèle de biomarqueur cible et les données de biomarqueurs pour la personne après avoir suivi le plan de repas recommandé pendant une période prédéterminée, et

un port de communication permettant de fournir le plan de repas recommandé à une interface utilisateur et d'afficher le plan de repas et l'indice de biomarqueur prédit à la personne pour sélection par la personne.

2. Système selon l'une quelconque des revendications précédentes, dans lequel le moteur d'inférence est également configuré pour (g) générer, sur la base des données de biomarqueurs, un indice de biomarqueur indiquant un alignement global entre le modèle de biomarqueur cible et les données de biomarqueurs pour la personne, et le port de communication est également destiné à fournir l'indice de biomarqueur à la personne.

3. Système selon la revendication 2, dans lequel l'indice de biomarqueur est basé sur une fonction pondérée d'écarts entre des niveaux de biomarqueurs cibles définis par le modèle de biomarqueur cible et des niveaux de biomarqueurs mesurés dans les données de biomarqueurs pour la personne.

4. Système selon la revendication 3, dans lequel la fonction pondérée comporte un coefficient de biomarqueur appliqué à l'écart pour chaque biomarqueur cible respectif, le coefficient de biomarqueur indiquant une importance relative du biomarqueur cible respectif par rapport à d'autres biomarqueurs dans le modèle de biomarqueur cible pour les un ou plusieurs médicaments.

5. Système selon l'une quelconque des revendications précédentes, dans lequel les données de santé comportent des données de biomarqueurs pour une pluralité de personnes à qui on a précédemment administré les un ou plusieurs médicaments et les nutriments correspondants consommés par la pluralité de personnes pendant la période correspondant à l'administration des un ou plusieurs médicaments.

6. Système selon l'une quelconque des revendications précédentes, dans lequel la pluralité de requêtes correspond à des effets secondaires courants associés aux un ou plusieurs médicaments.

7. Système selon l'une quelconque des revendications précédentes, dans lequel l'effet négatif correspond à un ou plusieurs effets secondaires indésirables qui ont un impact négatif sur l'efficacité des un ou plusieurs médicaments ou qui ont un impact négatif sur le respect d'un régime prescrit des un ou plusieurs médicaments.

8. Système selon la revendication 7, dans lequel les un ou plusieurs effets secondaires indésirables comportent au moins l'un du ballonnement, des gaz, de la nausée, du vomissement, de la diarrhée, de la fatigue, de la constipation, de la prise de poids, de la perte de poids et toute combinaison de deux des effets précédents ou plus.

9. Système selon l'une quelconque des revendications précédentes, dans lequel l'effet bénéfique correspond à un ou plusieurs effets thérapeutiques cibles des un ou plusieurs médicaments.

10. Système selon l'une quelconque des revendications précédentes, dans lequel le modèle de biomarqueur cible est déterminé sur la base de directives consensuelles concernant des plages de biomarqueurs cibles pour un individu en bonne santé, les informations démographiques de la personne et le traitement médical.

11. Système selon la revendication 10, dans lequel les informations démographiques comportent des informations génétiques obtenues sur la base d'un échantillon biologique de la personne.

12. Système selon l'une quelconque des revendications précédentes, dans lequel les une ou plusieurs bases de connaissances électroniques comprennent également (iv) des données cliniques liées à un contexte d'essai clinique, dans lequel le moteur d'inférence est également configuré pour remplacer une entrée de la personne par rapport aux un ou plusieurs médicaments si l'entrée est en conflit avec une ou plusieurs entrées dérivées des données cliniques.

13. Système selon l'une quelconque des revendications précédentes, comprenant également une interface utilisateur pour un clinicien associé à la personne, l'interface utilisateur comportant un tableau de bord présentant des options pour afficher des informations liées à la personne, comportant les informations démographiques de la personne, les données de santé liées aux données démographiques de la personne, les niveaux de biomarqueurs mesurés pour la personne, les un ou plusieurs médicaments à administrer à la personne, les quantités de nutriments dans la pluralité de repas précédemment consommés par la personne et le plan de repas recommandé.

14. Système selon l'une quelconque des revendications précédentes, comprenant également une interface utilisateur pour la personne, l'interface utilisateur comportant des options pour afficher des informations liées à la personne, comportant les informations démographiques de la personne, les niveaux de biomarqueurs mesurés pour la personne, les un ou plusieurs médicaments à administrer à la personne, les quantités de nutriments dans la pluralité de repas précédemment consommés par la personne et le plan de repas recommandé.

FIG. 1

<u>200</u>

```
┌─────────────────────────────────────────────────────────────────┐
│ 202 RECEIVE BIOMARKER DATA INDICATIVE OF A MEASURED BIOMARKER     │
│ LEVEL FOR EACH OF A PLURALITY OF BIOMARKERS FOR A PERSON          │
└─────────────────────────────────────────────────────────────────┘
                              │
                              ▼
┌─────────────────────────────────────────────────────────────────┐
│ 204 RECEIVE MEDICAL TREATMENT DATA INDICATIVE OF ONE OR MORE      │
│ MEDICATIONS TO BE ADMINISTERED TO THE PERSON                      │
└─────────────────────────────────────────────────────────────────┘
                              │
                              ▼
┌─────────────────────────────────────────────────────────────────┐
│ 206 DEDUCE, BASED ON THE MEDICAL TREATMENT DATA, THE BIOMARKER    │
│ DATA, HEALTH DATA, AND MEDICAL DATA, A PLURALITY OF QUERIES       │
│ ASSOCIATED WITH A PLURALITY OF EFFECTS OF THE ONE OR MORE         │
│ MEDICATIONS                                                       │
└─────────────────────────────────────────────────────────────────┘
                              │
                              ▼
┌─────────────────────────────────────────────────────────────────┐
│ 208 RECEIVE A PLURALITY OF RESPONSES TO THE QUERIES               │
└─────────────────────────────────────────────────────────────────┘
                              │
                              ▼
┌─────────────────────────────────────────────────────────────────┐
│ 210 GENERATE, BASED ON THE RESPONSES, A RECOMMENDED MEAL PLAN BY  │
│ MODIFYING A CANDIDATE NUTRIENT TEMPLATE SELECTED FROM A PLURALITY │
│ OF NUTRIENT TEMPLATES, WHEREIN THE MODIFYING IS BASED ON A        │
│ PLURALITY OF PREVIOUSLY CONSUMED MEALS BY THE PERSON, AND THE     │
│ RECOMMENDED MEAL PLAN IS DESIGNED TO REDUCE A NEGATIVE EFFECT OF  │
│ THE ONE OR MORE MEDICATIONS, INCREASE A BENEFICIAL EFFECT OF THE  │
│ ONE OR MORE MEDICATIONS AND/OR MINIMIZE A DEVIATION FROM A        │
│ TARGET BIOMARKER TEMPLATE FOR THE PERSON                          │
└─────────────────────────────────────────────────────────────────┘
```

FIG. 2

## 300

```
┌─────────────────────────────────────────┐
│ 320 RECEIVE A FIRST INPUT INDICATIVE OF  │
│ BIOMARKER DATA ACQUIRED FROM A GROUP OF  │
│ PEOPLE BEFORE A TIME PERIOD              │
└─────────────────────────────────────────┘
                    │
                    ▼
┌─────────────────────────────────────────┐
│ 322 RECEIVE MULTIPLE CANDIDATE NUTRIENT  │
│ TEMPLATES                                │
└─────────────────────────────────────────┘
                    │
                    ▼
┌─────────────────────────────────────────┐
│ 324 ASSIGN A SUBSET OF THE GROUP OF PEOPLE│
│ TO ONE OF THE CANDIDATE NUTRIENT         │
│ TEMPLATES FOR NUTRIENT CONSUMPTION       │
│ DURING THE TIME PERIOD                   │
└─────────────────────────────────────────┘
                    │
                    ▼
┌─────────────────────────────────────────┐
│ 326 RECEIVE A SECOND INPUT INDICATIVE OF │
│ BIOMARKER DATA ACQUIRED FROM THE GROUP   │
│ OF PEOPLE AFTER THE TIME PERIOD          │
└─────────────────────────────────────────┘
                    │
                    ▼
┌─────────────────────────────────────────┐
│ 328 FOR EACH PERSON, ASSESS A FIRST      │
│ BIOMARKER INDEX REPRESENTATIVE OF AN     │
│ ALIGNMENT BETWEEN THE BIOMARKER DATA IN  │
│ THE FIRST INPUT AND A RECOMMENDED        │
│ GUIDELINE AND A SECOND BIOMARKER INDEX   │
│ REPRESENTATIVE OF AN ALIGNMENT BETWEEN   │
│ THE BIOMARKER DATA IN THE SECOND INPUT AND│
│ THE RECOMMENDED GUIDELINE                │
└─────────────────────────────────────────┘
                    │
                    ▼
┌─────────────────────────────────────────┐
│ 330 SELECT THE NUTRIENT TEMPLATE         │
│ ASSOCIATED WITH THE SUBSET OF THE GROUP  │
│ OF PEOPLE THAT EXHIBITED THE LARGEST     │
│ IMPROVEMENT IN ALIGNMENT TO THE          │
│ RECOMMENDED GUIDELINE                    │
└─────────────────────────────────────────┘
```

## FIG. 3

## INDEX FUNCTION 400

420

INDEX FUNCTION
CALL RECEIVED?

YES

422 IDENTIFY
USER

424 HAS INDEX
BEEN CALCULATED
SINCE LAST
ENTRY?

YES

438 RETRIEVE
INDEX FROM
MEMORY

440 RETURN INDEX
AND EXIT

NO

426 RETRIEVE USER
TARGET VECTOR TV

428 RETRIEVE USER
MEASURED VECTOR MV

430 RETRIEVE USER
WEIGHT VECTOR WV

434 COMPUTE PRODUCT
WV*(TV-MV) FOR EACH
BIOMARKER

436 SUM ALL PRODUCTS TO
OBTAIN INDEX

## FIG. 4

**500**

EP 3 497 601 B1

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9011153 B
- US 2012130732 A1